# EUROPEAN PATENT APPLICATION

(11) **EP 3 875 128 A1**
(43) Date of publication of application: **08.09.2021**
(21) Application number: 21170272.5
(22) Date of filing: 23.09.2015
(51) Int. Cl.: A61M 5/142, A61M 5/162, A61J 1/10, A61M 5/168

(54) **DEVICES A FOR DELIVERING A BENEFICIAL AGENT TO A USER**

(30) Priority: 23.09.2014 US 201462054146 P
(62) Divisional of application: 15775882.2
(71) Applicant: AbbVie Inc., North Chicago, IL 60064 (US)
(72) Inventor: HANAGAN, Ted, Libertyville (US); KLINGLER, Wayne, Lindenhurst (US); GRAZIER, Thomas, Spring Grove (US); DHAMI, Gurjinder, Neenah (US); SMIEJA, Scott, Oshkosh (US); SCHACHERL, Jeff, Neenah (US); MACKEY, Sean, Grayslake (US); FEILEN, Megan, Franklin (US); ZHOU, Ji, Lake Villa (US); CONJEEVARAM, Rajkumar, Lake Bluff (US); GIBLER, Martin, West Chester (US)
(74) Representative: Elkington and Fife LLP

(57) **Abstract**

Drug delivery reservoir for delivery of a beneficial agent to a user includes a drug delivery reservoir housing (10) having a fluid reservoir (12) defined therein. The drug delivery reservoir housing (10) has a drug delivery reservoir base region (83). The drug delivery reservoir includes a dip tube (13) extending inside the fluid reservoir. The dip tube (13) includes a tubular wall defining a flow lumen. The tubular wall has at least one aperture (14) defined therein and spaced proximally from a distal end of the tubular wall in fluid communication with the fluid reservoir. The drug delivery reservoir includes an adaptor (15) disposed external to the drug delivery reservoir housing (10) and coupled to a proximal end of the dip tube.

## Description

### CROSS-REFERENCE TO RELATED APPLICATIONS

This application claims priority to U.S. Provisional Patent Application No. 62/054,146, filed September 23, 2014, which is incorporated by reference herein in its entirety.

### BACKGROUND

### Field of the Disclosed Subject Matter

The disclosed subject matter relates to devices, systems and methods for controlling and delivering fluids, for example for delivery of a beneficial agent to a user.

### Description of Related Art

The disclosed subject matter is generally related to devices, systems and methods for controlling and delivering fluids, for example for delivery of a beneficial agent to a user.

A variety of fluid transport devices and systems have been developed for controlling and delivering beneficial agents in fluid form. Such fluid flow systems can include 1) volumetric-based aspiration flow systems using positive displacement pumps, and 2) vacuum-based aspiration systems using a vacuum source. For example, volumetric aspiration systems include peristaltic pumps for the delivery of therapeutic agents to a user. Various forms of peristaltic pumps are known, such as using rotating rollers to press against a flexible tubing to induce flow therethrough. Cassette systems or other drug delivery reservoir configurations can be coupled with the pump device to provide a source of beneficial agent fluid via the flexible tubing.

Such devices and systems are particularly beneficial as portable infusion pumps capable of being worn or carried by the user. However, there remains a need for improvement of such devices and systems. For example, it is desirable to deliver a generally uniform concentration of beneficial agent throughout the delivery process. However, it is possible the concentration of beneficial agent is not or will not remain uniform throughout the fluid reservoir. As such, there is a need and desire for a drug delivery reservoir capable of providing more uniform delivery of the beneficial agent throughout the delivery process.

### SUMMARY

The purpose and advantages of the disclosed subject matter will be set forth in and apparent from the description that follows, as well as will be learned by practice of the disclosed subject matter. Additional advantages of the disclosed subject matter will be realized and attained by the methods and systems particularly pointed out in the written description and claims hereof, as well as from the appended drawings.

To achieve these and other advantages and in accordance with the purpose of the disclosed subject matter, as embodied and broadly described, the disclosed subject matter includes a drug delivery reservoir for delivery of a beneficial agent to a user. The drug delivery reservoir generally includes a drug delivery reservoir housing, a dip tube and an adaptor. The drug delivery reservoir housing has a fluid reservoir defined therein and a drug delivery reservoir base region. The dip tube extends inside the fluid reservoir and includes a tubular wall defining a flow lumen. The tubular wall has at least one aperture defined therein and spaced proximally from a distal end of the tubular wall in fluid communication with the fluid reservoir. The adaptor is disposed external to the drug delivery reservoir housing and coupled to a proximal end of the dip tube.

Additionally, and as embodied herein, the fluid reservoir can be a flexible bag disposed within the housing. In some embodiments, the dip tube can be disposed diagonally across an interior region of the fluid reservoir. Additionally or alternatively, the dip tube can be disposed along a perimeter of the fluid reservoir, or at least a portion of the dip tube can be disposed proximate a center region.

Furthermore, and as embodied herein, the tubular wall can have a plurality of apertures spaced apart along a length of the tubular wall. One of the plurality of apertures nearest the outlet end can spaced from the outlet end a distance of at least 15% of the length of the tubular wall. In some embodiments, one of the plurality of apertures nearest the outlet end is spaced from the outlet end a distance of about 20% of the length of the tubular wall.

In addition, and as embodied herein, the plurality of apertures can be configured to provide a generally uniform distribution of flow through the plurality of apertures along the length of the tubular member. The plurality of apertures can vary in spacing between adjacent apertures along the length of the tubular wall. In some embodiments, the plurality of apertures can decrease in spacing toward the distal end of the tubular wall. Additionally or alternatively, the plurality of apertures can vary in cross dimension along the length of the tubular wall. In some embodiments, the plurality of apertures can increase in cross dimension along the length of the tubular wall. For example, and as embodied herein, a size of the plurality of apertures can increase along the tubular wall from the outlet end toward the distal end.

Additionally, and as embodied herein, the plurality of apertures can have a slotted shape. Alternatively, the plurality of apertures can have a circular shape. At least two of the plurality of apertures can be aligned axially along the length of the tubular wall and spaced circumferentially about the tubular wall. Additionally or alternatively, at least three of the plurality of apertures are aligned axially along the length of the tubular wall and spaced circumferentially about the tubular wall.

Furthermore, and as embodied herein, the drug delivery reservoir can include fluid beneficial agent in the reservoir. The concentration of the beneficial agent may be generally uniform throughout the reservoir, or may be non-uniform. For example, the fluid beneficial agent can have a volume and a concentration increasing from a region proximate the outlet end to a region proximate the distal end. The dip tube can be configured to deliver the volume of the fluid beneficial agent at a substantially uniform concentration.

In some embodiments, the drug delivery reservoir can include a junction with a first dip tube section and a second dip tube section each extending from an outlet thereof.

It is to be understood that both the foregoing general description and the following detailed description are exemplary and are intended to provide further explanation of the disclosed subject matter claimed.

The accompanying drawings, which are incorporated in and constitute part of this specification, are included to illustrate and provide a further understanding of the disclosed subject matter. Together with the description, the drawings serve to explain the principles of the disclosed subject matter.

### BRIEF DESCRIPTION OF THE DRAWINGS

FIG. 1 is an exploded plan view of an exemplary device for delivering a beneficial agent according to the disclosed subject matter.
FIG. 2 is a perspective view of the device of FIG. 1.
FIG. 3 is a plan view of an exemplary fluid reservoir and delivery tube assembly of the disclosed subject matter.
FIG. 4 is a plan view of another embodiment of a fluid reservoir and delivery tube assembly of the disclosed subject matter.
FIG. 5A is a schematic view of another embodiment of a fluid reservoir and delivery tube assembly of the disclosed subject matter.
FIG. 5B is a schematic view of another embodiment of a fluid reservoir and delivery tube assembly of the disclosed subject matter.
FIG. 5C is a schematic view of an exemplary fluid reservoir of the disclosed subject matter.
FIG. 5D is a plan view of yet another embodiment of fluid reservoir and delivery tube assembly of the disclosed subject matter.
FIG. 5E is a schematic view of yet another embodiment of fluid reservoir and delivery tube assembly of the disclosed subject matter.
FIGS. 6A-6B are top-left perspective and rear-right perspective views, respectively, of an exemplary drug delivery reservoir of the device of FIG. 1.
FIG. 7 is a perspective view of another exemplary device for delivering a beneficial agent according to the disclosed subject matter, with the cassette separated from the pump.
FIGS. 8-11 each sequentially shows the cassette and pump of FIG. 7 being joined, with a latch in an open position.
FIG. 12 shows the cassette and pump of FIG. 7 joined with the latch in a closed position.
FIGS. 13A-13C together illustrate an exemplary embodiment of an aperture configuration, which can be used with any of the dip tubes according to the disclosed subject matter.
FIGS. 14A-14D together illustrate another exemplary embodiment of an aperture configuration, which can be used with any of the dip tubes according to the disclosed subject matter.
FIG. 15 illustrates another exemplary embodiment of an aperture configuration, which can be used with any of the dip tubes according to the disclosed subject matter.
FIGS. 16A-16D together illustrate yet another exemplary embodiment of an aperture configuration, which can be used with any of the dip tubes according to the disclosed subject matter.
FIG. 17 is a diagram illustrating additional details of an exemplary peristaltic tubing according to the disclosed subject matter.
FIG. 18A is a left side view of an exemplary junction fitting according to the disclosed subject matter.
FIG. 18B is cross-sectional side view taken along line A-A of FIG. 18A.
FIG. 18C is an enlarged cross-sectional view of region C of FIG. 18B.
FIG. 18D is a plan view of the junction fitting of FIG. 18A.
FIG. 19 is a diagram illustrating exemplary beneficial agent concentration per dispensed volume for drug delivery reservoirs using exemplary dip tube configurations according to the disclosed subject matter compared to cassettes using no dip tube.
FIG. 20 is a diagram illustrating exemplary beneficial agent concentration per dispensed volume for drug delivery reservoirs using exemplary dip tube configurations according to the disclosed subject matter.
FIG. 21 is a diagram illustrating exemplary beneficial agent concentration per dispensed volume for drug delivery reservoirs using exemplary dip tube configurations according to the disclosed subject matter.
FIG. 22 is a diagram illustrating exemplary beneficial agent concentration per dispensed volume for drug delivery reservoirs using exemplary dip tube configurations according to the disclosed subject matter.
FIG. 23 is a diagram illustrating an exemplary beneficial agent concentration for a plurality of regions of a fluid according to the disclosed subject matter.

### DETAILED DESCRIPTION

Reference will now be made in detail to the exemplary embodiments of the disclosed subject matter, examples of which are illustrated in the accompanying drawings. The methods of the disclosed subject matter will be described in conjunction with the detailed description of the system. The devices and methods presented herein can be used for delivering a beneficial agent to a user.

The accompanying figures, where like reference numerals refer to identical or functionally similar elements throughout the separate views, serve to further illustrate various embodiments and to explain various principles and advantages all in accordance with the disclosed subject matter.

The apparatus and methods presented herein can be used for administering any of a variety of suitable therapeutic agents or substances, such as a drug or biologic agent, to a patient. For example, and as embodied herein, a drug delivery reservoir is provided for use with a pump or the like to deliver a beneficial agent to a user. The drug delivery reservoir includes a housing having a fluid reservoir defined therein. The housing can be in the form of a cassette or similar rigid body. The fluid reservoir containing a fluid substance can be joined to a delivery tube system. In operation, the pump can operate on the drug delivery reservoir to deliver the fluid substance through the tubing system. In this manner, the device is capable of administering a dosage of the fluid substance, such as a therapeutic agent, including a formulation in a liquid or gel form, through the delivery tube system and to a patient. In some embodiments, the fluid therapeutic agent can include one or more pharmaceutical or biologic agents.

In accordance with the disclosed subject matter, a drug delivery reservoir for delivery of a beneficial agent to a user is provided. The drug delivery reservoir generally includes a drug delivery reservoir housing having a fluid reservoir defined therein. The drug delivery reservoir housing includes a drug delivery reservoir base region. The drug delivery reservoir includes a dip tube extending inside the fluid reservoir. The dip tube includes a tubular wall defining a flow lumen. The tubular wall can include at least one aperture defined therein and spaced proximally from a distal end of the tubular wall in fluid communication with the fluid reservoir. The drug delivery reservoir further includes an adaptor coupled to a proximal end of the dip tube. The adaptor can be disposed external to the drug delivery reservoir housing.

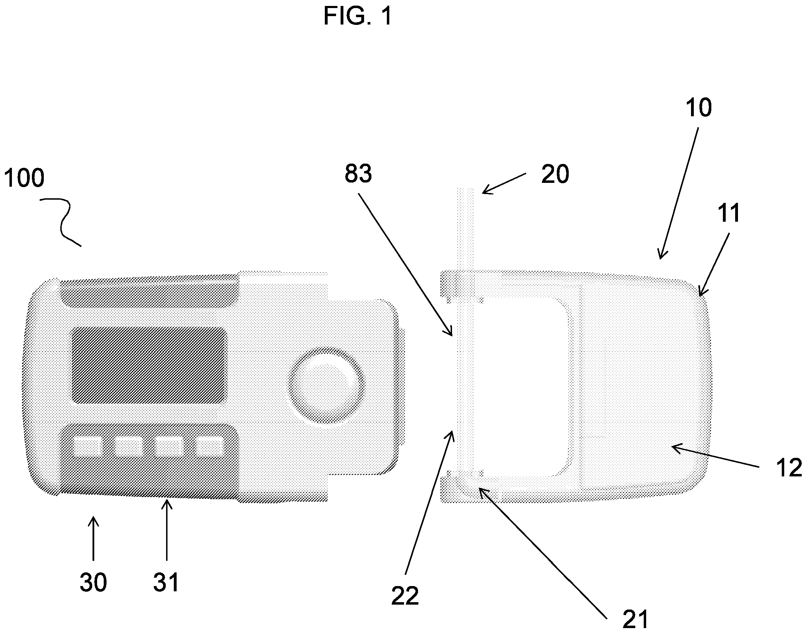

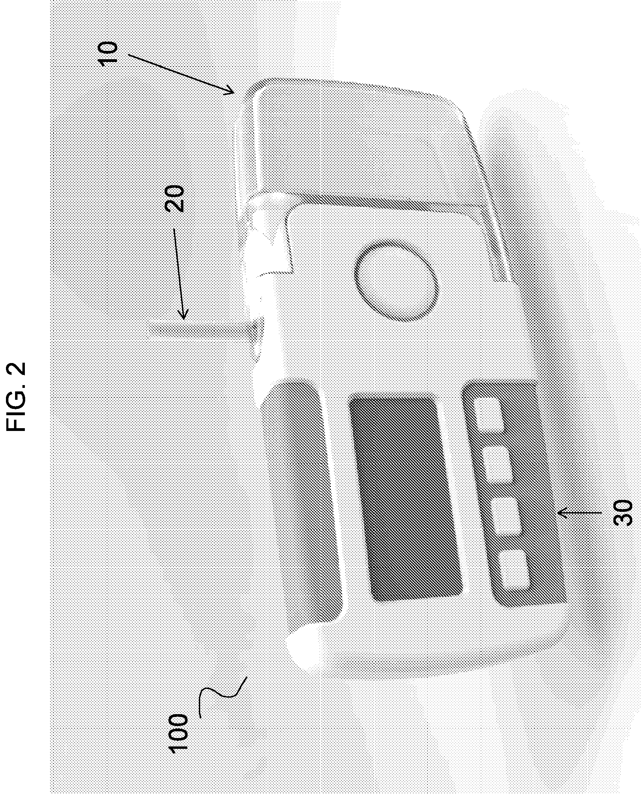

For the purpose of explanation and illustration, and not limitation, an exemplary embodiment of the device in accordance with the disclosed subject matter is shown in Figs. 1-2 and is designated generally by reference character 100. As embodied herein, for purpose of illustration and not limitation, the device 100 is provided in the form of a cassette 10. The cassette 10 has a cassette housing 11 and a fluid reservoir 12 defined within the cassette housing 11. The cassette housing 11 can include a cassette base region 83 to join with the pump mechanism 30 having a pump housing 31, as discussed further herein. As shown in Figs. 1 and 2, the device can also include a delivery tube 20, as discussed further herein. In some embodiments, the delivery tube 20 can have a first portion 21 disposed within the cassette housing 11 and second portion 22 disposed outside the cassette housing 11. In this manner, the housing 11 is a rigid member. Alternatively, the housing itself can be in the form of a flexible pouch or the like to define the fluid reservoir 12.

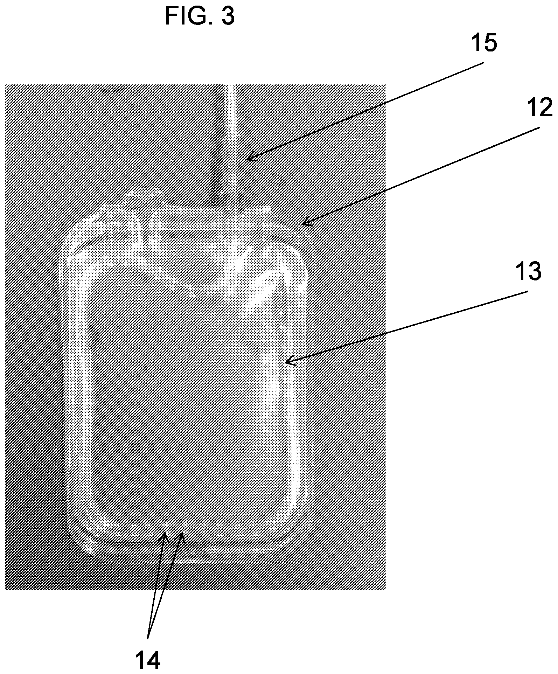

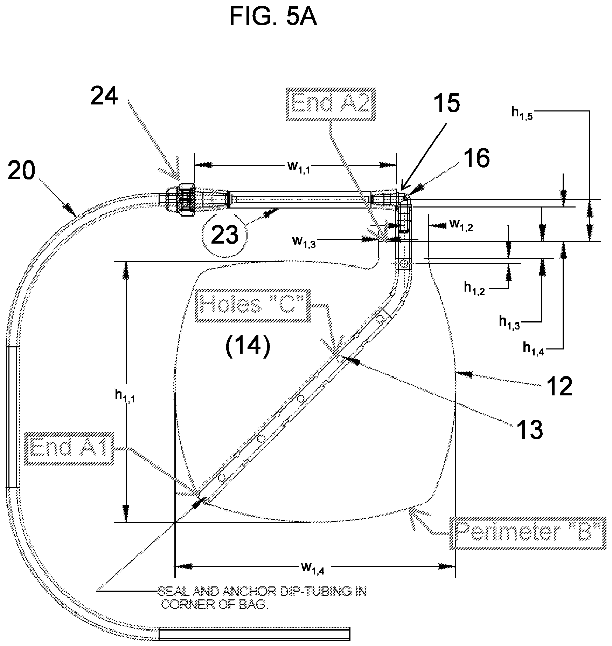

In accordance with the disclosed subject matter, the fluid reservoir 12 can be defined by the interior surface of the cassette housing 11. Alternatively, as depicted here, the fluid reservoir 12 can be defined by a separate member disposed inside the cassette housing 11. For example, the fluid reservoir 12, shown in the various embodiments of FIGS. 3, 4, and 5A-C, respectively, for the purpose of illustration and not limitation, can be configured as a flexible pouch. The fluid reservoir 12 of each embodiment can also have a textured inner surface as described further below. Opposing sides of the pouch can be secured about a perimeter (such as denoted by "Perimeter B" in FIG. 5A) to form the fluid reservoir 12, for example by thermal or radio frequency (RF) welding or the like. The fluid reservoir 12 can have a "rounded square shape." As shown for example in Figs. 3-5E, and as embodied herein, the fluid reservoir 12 can be generally square-shaped with rounded corners. The rounded corners can allow the bag to fit more easily into the cassette housing 11, allow the bag to fill more evenly with a fluid beneficial agent, and inhibit or prevent the fluid beneficial agent from becoming trapped or otherwise unable to be removed from the fluid reservoir during normal operation of the cassette and pump. Additionally or alternatively, ridges can be formed on the surface of the fluid reservoir 12. The ridges can allow the fluid beneficial agent to be more easily drawn into the tube.

The fluid reservoir 12 can be formed from a flexible material having low oxygen permeability. For purpose of illustration and not limitation, the fluid reservoir 12 can be made of EVA/EVOH/EVA, TOTM Plasticized PVC, combinations thereof, or other suitable materials, and as embodied herein, can be made of Renolit Solmed® Medipak UVO 9002. For purpose of illustration and not limitation, as embodied herein, the fluid reservoir 12 can have a thickness of about 12 mil. Additionally, for purpose of illustration and not limitation, the fluid reservoir 12 can be formed using an adhesive, by RF welding, or any other suitable technique.

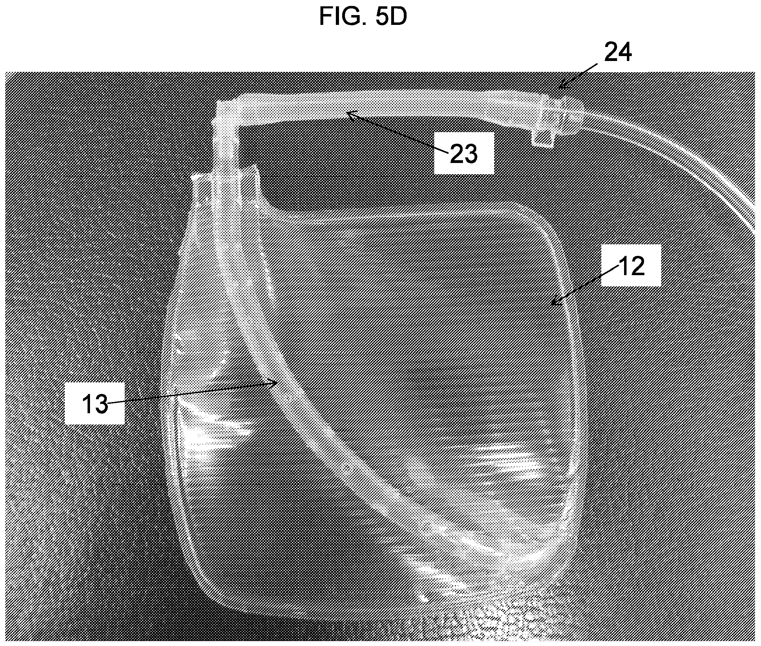

As described herein, and in accordance with the disclosed subject matter, a dip tube 13 is disposed inside the fluid reservoir 12. The dip tube 13 includes a tubular wall 13a defining a flow lumen. The tubular wall 13a of the dip tube 13 disclosed herein can have at least one aperture 14 defined therein and be spaced proximally from a distal end of the tubular wall 13a. The aperture 14 is in fluid communication with the reservoir 12 to receive a beneficial agent contained within the reservoir 12. Furthermore, the inner surface of the fluid reservoir 12 can have a textured, ribbed or grooved configuration to further enhance fluid flow by preventing unintended occlusion of the apertures 14. For example, and as embodied herein, fluid reservoir 12 can include a plurality of horizontal grooves formed therein, as shown in FIG. 5D.

In accordance with an additional aspect of the disclosed subject matter, dip tube 13 can include a plurality of apertures 14, as shown for example in FIG. 5A. For example, and as embodied herein, the plurality of apertures 14 each can be the same or similar size with the same or similar spacing therebetween. In accordance with the disclosed subject matter, the plurality of apertures can be configured to provide generally uniform flow distribution through the apertures along a length of the tubular wall of the dip tube. For example, and as described further below, the plurality of apertures 14 can have different sizes and/or have uneven distribution along the length of the dip tube 13. Various combinations of variations in aperture size, shape and/or spacing along the tubular wall are described below for purpose of illustration and not limitation. For purpose of illustration, and not limitation, as embodied herein, apertures can be formed by machining, laser perforation or any other suitable techniques.

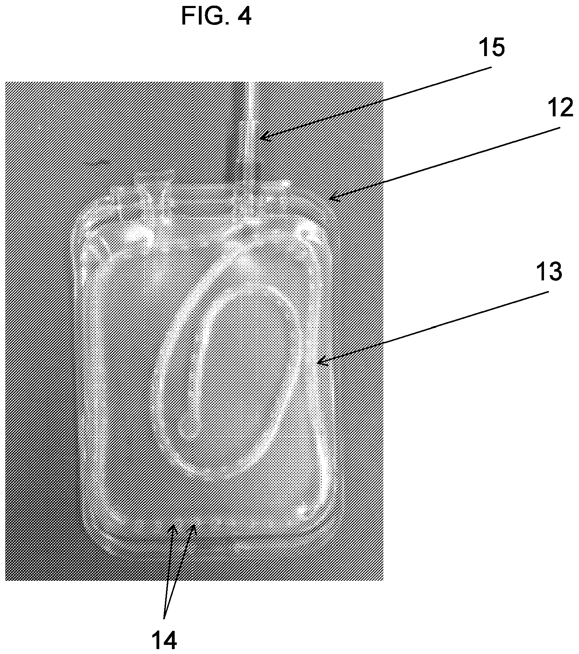

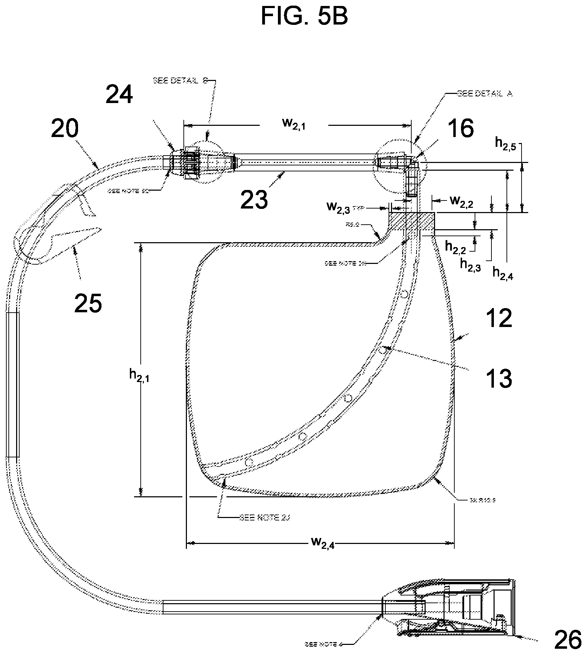

Generally, the dip tube is configured to bridge or otherwise extend at least through the area expected to have the highest concentration of beneficial agent within the fluid reservoir. For purpose of illustration and not limitation, and as embodied herein, the dip tube 13 can be arranged in any of a number of suitable configurations within the fluid reservoir 12. For example, and as shown in FIG. 3, the dip tube 13 can extend along the perimeter of the fluid reservoir 12. Additionally or alternatively, the dip tube 13 can be coiled within a central region of the fluid reservoir 12, as depicted in FIG. 4. In addition, or as a further alternative, the dip tube 13 can form a serpentine configuration within all or a portion of the fluid reservoir 12. In accordance with yet another embodiment, as shown in FIG. 5A, the dip tube 13 can extend diagonally across the fluid reservoir 12 from one extreme end or corner to another. In accordance with yet another embodiment, as shown in FIG. 5B, the dip tube 13, can extend diagonally across the fluid reservoir 12 from one extreme end or corner to another, and having a bend to define an arcuate shape therebetween.

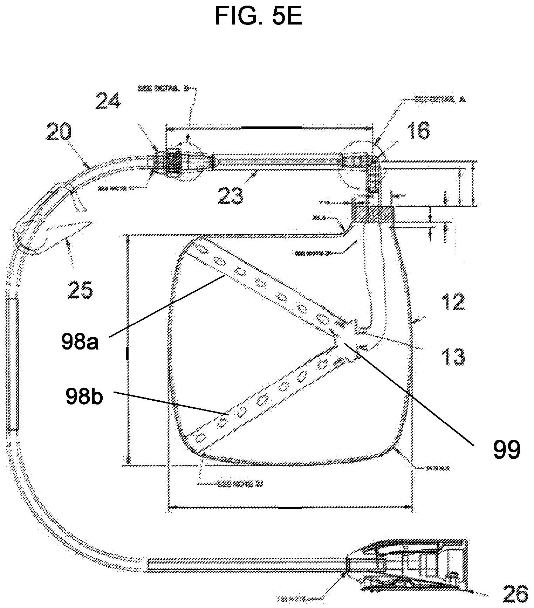

In accordance with yet another embodiment, as shown in FIG. 5E, the dip tube 13 can extend to a junction 99 disposed within the reservoir 12 with dip tube sections 98a, 98b extending from the junction 99. For purpose of illustration and not limitation, and as embodied herein, dip tube 13 can be free of apertures between the outlet end 33 and the junction 99. Alternatively, dip tube 13 can include one or more apertures between the outlet end 33 and the junction 99 in any configuration described herein. Sections 98a, 98b can extend from the junction 99, for example and as embodied herein, with a first section 98a extending toward an upper region of the reservoir 12 and a second section 98b extending toward a lower region of the reservoir 12 relative the outlet. As embodied herein, sections 98a, 98b each can include one or more apertures in any configuration described herein. For example and as embodied herein, sections 98a, 98b can include similar aperture configurations. Alternatively, sections 98a, 98b each can include varied aperture configurations. For purpose of illustration and not limitation, as embodied herein, the upper and lower regions can have different concentrations of a beneficial agent, and as such, having a dip tube 13 with sections 98a, 98b disposed in the upper and lower regions can be configured to allow different concentrations of a beneficial agent to be drawn from the reservoir through the different apertures 14 at substantially the same time for an overall more uniform concentration of beneficial agent delivered from the device during the delivery process.

In operation, the perforated dip tube 13 of each embodiment according to the disclosed subject matter allows fluid to be drawn from the fluid reservoir 12 regardless of the orientation of the reservoir 12. For example, and with reference to FIG. 3, the dip tube 13 can be disposed generally along the perimeter of the reservoir 12, which can include placing the dip tube 13 proximate the "corners" of the reservoir 12, if provided. Additionally or alternatively, for example as shown in FIG. 4, the dip tube 13 can have one or more portions disposed proximate the center of the reservoir 12. In this manner, if liquid becomes trapped in the center of the reservoir 12, for example if the reservoir 12 becomes oriented horizontally, the dip tube 13 can receive the fluid from the center of the reservoir 12.

Referring now to FIGS. 5A and 5B, for illustration and not limitation, the dip tube 13 embodied herein for use within a fluid reservoir as shown can be configured using SUNLITE VYSUN 102-80-26 (Non-DEHP PVC) tube material, resin material such as Dupont Elvax 3182-2 EVA or silicone tube material, for example Saint-Gobain's Biosil Precision silicone tubing. As embodied herein, for a fluid reservoir 12 having a height of approximately 80 inches and a width of approximately 74 inches, the dip tube 13 can have a length of approximately 105 mm, and the tubular wall 13a can have a plurality of approximately 2 mm diameter apertures (denoted as "Holes C" in FIG. 5A) disposed therein. As shown for example in FIG. 5A, apertures C can be disposed along the tubular wall 13a of the dip tube 13 starting from a location 8.5 mm from the tube interior or distal end (denoted as "End A1" in FIG. 5A). The distal end of the dip tube 13 can have any suitable size or shape. The distal end of the dip tube 13 can be closed to ensure all fluid flow is through the apertures 14. For example and without limitation, the distal end of the dip tube 13 can be flattened, tapered, or flared. As embodied herein, each aperture C can be spaced apart 8 mm along the length of the tubular wall 13a of dip tube 13 and rotated 90 degrees about the tubular wall 13a of dip tube 13 relative to adjacent apertures C. Representative dimensions of exemplary fluid reservoir and dip tube assemblies, for purpose of illustration and not limitation, are set forth below.

| **FIG.5A** | **Exemplary Dimensions (mm)** |
|---|---|
| W_{1,1} | 58 |
| W_{1,2} | 7.165 |
| W_{1,3} | 1.6 |
| W_{1,4} | 80.2 |
| h_{1,1} | 74.2 |
| h_{1,2} | 1.5 |
| h_{1,3} | 4.78 |
| h_{1,4} | 9.9 |
| h_{1,5} | 12 |

| **FIG. 5B** **&** **5E** | **Exemplary Dimensions (mm)** |
|---|---|
| W_{2,1} | 63.34±1.60 |
| W_{2,2} | 5.6±0.4 |
| W_{2,3} | 0.8±0.4 |
| W_{2,4} | 74.6 |
| h_{2,1} | 70.6 |
| h_{2,2} | 1.5 |
| h_{2,3} | 4.8 |
| h_{2,4} | 11.9 |
| h_{2,5} | 14 |

| **FIG. 5C** | **Exemplary Dimensions (mm)** |
|---|---|
| W_{3,1} | 4.6 |
| W_{3,2} | 5.6 |
| W_{3,3} | 0.8 |
| W_{3,4} | 74.6 |
| h_{3,1} | 70.6 |
| h_{3,2} | 4.8 |
| h_{3,3} | 11.9 |

Furthermore, and as embodied herein, the dip tube 13 can have an inside diameter of 3 mm and an outside diameter of 4.6 mm. In some embodiments, the dip tube 13 can have a thickness of at least about 1.5 mm; in some embodiments, the dip tube 13 can have a thickness of at least about 1.6 mm. As shown for example in FIG. 5A, the dip tube 13 can be disposed diagonally across the interior of the fluid reservoir 12 (i.e., bisecting fluid reservoir 12). In accordance with yet another embodiment, as shown in FIG. 5B, the dip tube 14, can extend diagonally across the fluid reservoir 12 from one corner or end to another, having a bend to define an arcuate shape therebetween. The dip tube 13 can be joined to the reservoir 12 at a reservoir entry port (denoted as "End A2" in FIG. 5A) as well as at the opposing end of the tube A1 inside the reservoir 12. As such, the dip tube 13 can be inhibited or prevented from movement within the reservoir 12.

The dip tube 13 can extend from the fluid reservoir 12 to serve as a delivery tube if desired or appropriate. Alternatively, and as embodied herein, an adaptor disposed external to the cassette housing 11 can be provided and coupled to a proximal end of the dip tube 13. In this manner, a separate delivery tube can be coupled to the adaptor for delivery of the beneficial agent from the fluid reservoir 12 to the user due to operation of the pump 30. Additionally, a peristaltic tube can be provided between or as a part of the dip tube 13 and/or the delivery tube for interaction with the pump 30.

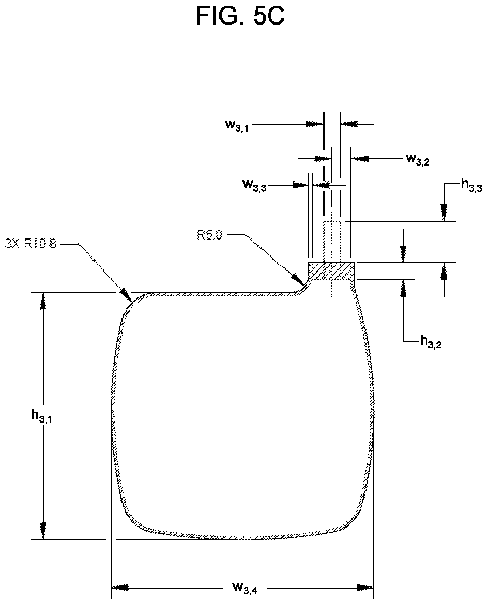

For the purpose of illustration and not limitation, exemplary embodiments of such an adaptor are depicted in FIGS. 5A-B. As shown, the fluid reservoir 12 includes an adaptor 15 disposed external to the cassette housing 11. The adaptor 15 of FIG. 5 is coupled to a proximal end of the dip tube 13. As embodied herein, a polypropylene-barbed elbow fitting 16 is provided at the proximal end of the dip tube 13. The elbow fitting 16 can be adhered to the exterior end of the dip tube 13 and oriented in plane with the fluid reservoir 12. A peristaltic tube 23 can be installed or coupled to an opposing end of the elbow fitting 16. For example, and as embodied herein, the peristaltic tube 23 can be formed from a section of Saint Gobain Biosil Precision PCS-Silicone tubing material. The peristaltic tube 23 can have an inside diameter of 1.6 mm and an outside diameter of 4.8 mm. A junction fitting 24 is joined to the peristaltic tube 23, and a delivery tube 20 can be adhered into the junction fitting 24. As such, the delivery tube 20 can be fluidly coupled with the fluid reservoir 12. For example and without limitation, , the delivery tube 20 can be formed from any suitable polymeric material or combination of materials, and as embodied herein, with an inner diameter of Dupont Elvax 3182-2 EVA and an outer diameter Colorite 8088G-015 Non-DEHP PVC.

A device having a fluid reservoir 12 and dip tube 13 as disclosed in FIGS. 5A-5B thus ensures delivery of a significant portion of beneficial agent regardless of the orientation of the fluid reservoir 12. Additionally, the use of a plurality of apertures reduces the risk associated with one or more apertures becoming occluded during delivery.

However, it has been determined that certain formulations of beneficial agent may result in non-uniform flow distribution through the apertures, such as when more viscous fluids are used (e.g., oils, gels or the like). As such, and in accordance with another aspect of the disclosed subject matter, dosing accuracy can be further enhanced by modifying the dip tube to increase uniformity of the amount of fluid uptake along the length of the dip tube. That is, in vacuum pump systems or the like, pressure can be lost between the vacuum supply point (e.g., in a peristaltic pump system, the interface between the pump fingers and the tube) and the fluid supply point, causing a change in pressure along the tubing of a vacuum pump system. Such a pressure loss is exacerbated with more viscous fluids, such as oils and gels, due to frictional and shear forces of the fluid through the relatively small tube. The change in pressure along the length of dip tube 13 can cause different amounts of fluid uptake along the length of dip tube 13 due to the plurality of apertures 14 along the length of dip tube 13. As such, and as disclosed herein, the plurality of apertures can be configured to provide a generally uniform distribution of flow through the plurality of apertures along the length of the tubular member. For example, apertures 14 disposed closer to the reservoir 12 outlet, where vacuum pressure is greatest, can be reduced in size, can be removed, and/or can be spaced further away from the outlet. In some embodiments, a number of apertures 14 spaced closer to the reservoir 12 outlet can be reduced. Additionally or alternatively, apertures 14 spaced further away from the reservoir 12 outlet can be increased in size. As a further alternative, the shape of some or all of the apertures 14 along the length of the tube can be modified, for example to have a slotted shape.

Additionally, the spacing between adjacent apertures can be varied along the length of the tubular wall. For purpose of illustration and not limitation, as embodied herein, the plurality of apertures decrease in spacing toward the distal end of the tubular wall. Alternatively, the plurality of apertures can increase in spacing toward the distal end of the tubular wall.

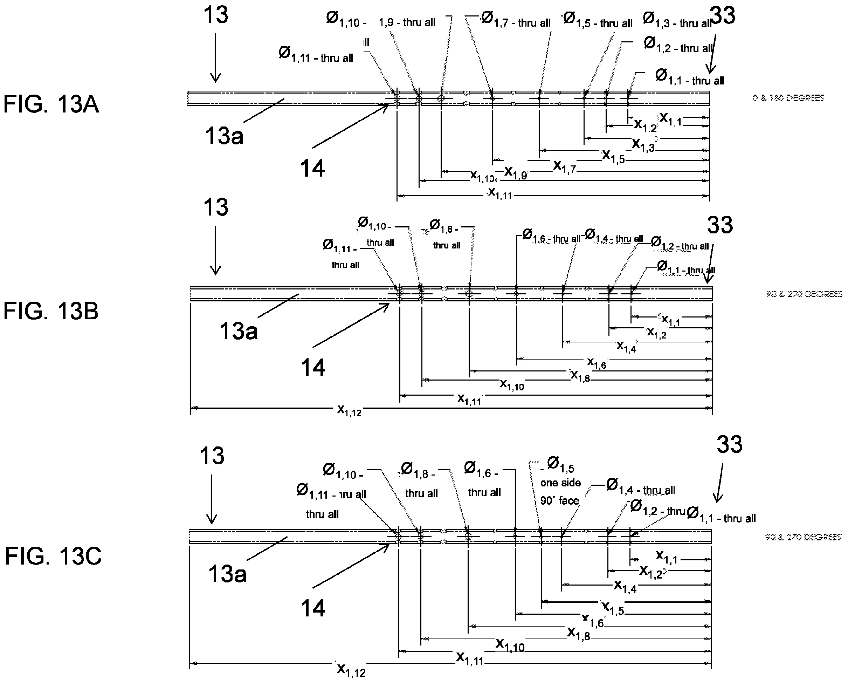

Furthermore, and as embodied herein, the plurality of apertures can vary in cross dimension along the length of the tubular wall. For purpose of illustration and not limitation, the size of apertures 14 can increase along the length of the dip tube 13 from the reservoir 12 outlet toward the end of the dip tube. As shown for example in FIGS. 13A-13C, dip tube 13 can have apertures 12 varying in size along the length of the dip tube, spaced apart from the outlet end 33 of the dip tube 13 by varying distances along the length of the tubular wall 13a of the dip tube 13, and rotated varying degrees about the tubular wall 13a of the dip tube 13. Increasing the size of apertures 14 along the length of the dip tube 13 from the outlet end 33 toward the distal end of the dip tube 13 can compensate for the decreasing vacuum pressure. For example, increasing the size of apertures 14 along the length of the dip tube 13 can result in a more uniform uptake of fluid along the dip tube 13.

Table 1 illustrates an exemplary dip tube aperture configuration. For purpose of illustration, and not limitation, hole number or hole location refers to an axial distance from the outlet end 33 of the dip tube 13, with the distance increasing as the hole number or location number increases. As embodied herein and illustrated in the following Tables, unless otherwise specified, hole number or location number 1 corresponds to an axial distance 18.18 mm from the outlet end 33 of the dip tube 13, and each successive hole number represents a distance of about an additional 8 mm from the outlet end 33 of the dip tube 13. As such, a fractional hole number or location number represents a fraction of the 8 mm spacing.

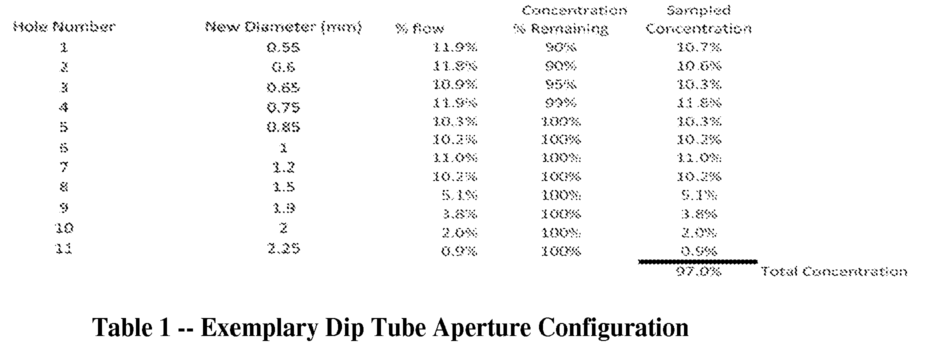

Table 2 illustrates another exemplary dip tube aperture configuration. As shown, no apertures were formed in the first two hole locations (e.g., spaced about 18.18 mm and 26.18 mm from the outlet end 33). As such, the first aperture was formed in hole location 3, spaced about 34.18 mm from the outlet end 33, which is about 20% of the length of the dip tube 13. Apertures were formed at 9 axial locations along the dip tube 13 and have a uniform diameter. For purpose of comparison and confirmation of the disclosed subject matter, as illustrated in Table 3, flow uniformity is improved over dip tube configurations having constant diameter apertures, uniform spacing, and apertures formed closer to the outlet end 33 of the dip tube 13. In this configuration, the initial aperture can be located in a region of relatively low concentration gradient, which can provide more uniform concentration of beneficial agent delivered during the delivery process.

For purpose of comparison with and confirmation of the disclosed subject matter, a representative formulation having a high viscosity and varied concentration was produced for purpose of illustration. For example and without limitation, the representative formulation was formed with Boron Nitride (BN) and a highly viscous gel, as embodied herein at a ratio of 6.77%(w/w) of Boron Nitride to the gel. The composition of the representative formulation is shown in Table A.

**Table A -- Representative Formulation Composition**

| **Ingredient** | **Lot # (Vendor)** | **Percent Weight** | **Theoretical Weight (g)** | **Actual Weight (g)** |
|---|---|---|---|---|
| Boron Nitride Powder | 3-5048-00-21 (ZYP Coatings) | 6.77 | 241.4 | 241.6 |
| NaCMC 2000 | 2C1550NEFC (Biogrund) | 1.58 | 56.21 | 56.5 |
| NaCMC 700 | 212250NEFA (Biogrund) | 1.29 | 45.99 | 46.1 |
| DI Water | N/A | 90.37 | 3222.8 | 3221.4 |
| Total | | 100 | 3566.4 | 3565.6 |

Sample fluid reservoirs, for example as illustrated in FIG. 5A and 5D, were filled with 50 mL of the representative formulation and assembled into a drug delivery reservoir. To further illustrate the effect of varied concentration within the fluid reservoir, the drug delivery reservoirs were installed on a centrifuge, which was operated for a duration of 66 hours to accelerate the BN within the gel to produce a varied concentration of BN throughout the gel. The operating conditions of the centrifuge are shown in Table B.

**Table B -- Centrifuge Operating Conditions**

| Radius (in) | Radius (m) | Frequency (Hz) | Speed (RPM) | Speed (rad/s) | Acceleration (m/s^2) | Relative centrifugal force (G's) |
|---|---|---|---|---|---|---|
| 36.5 | 0.93 | 2.04 | 122.4 | 12.82 | 152.32 | 15.53 |

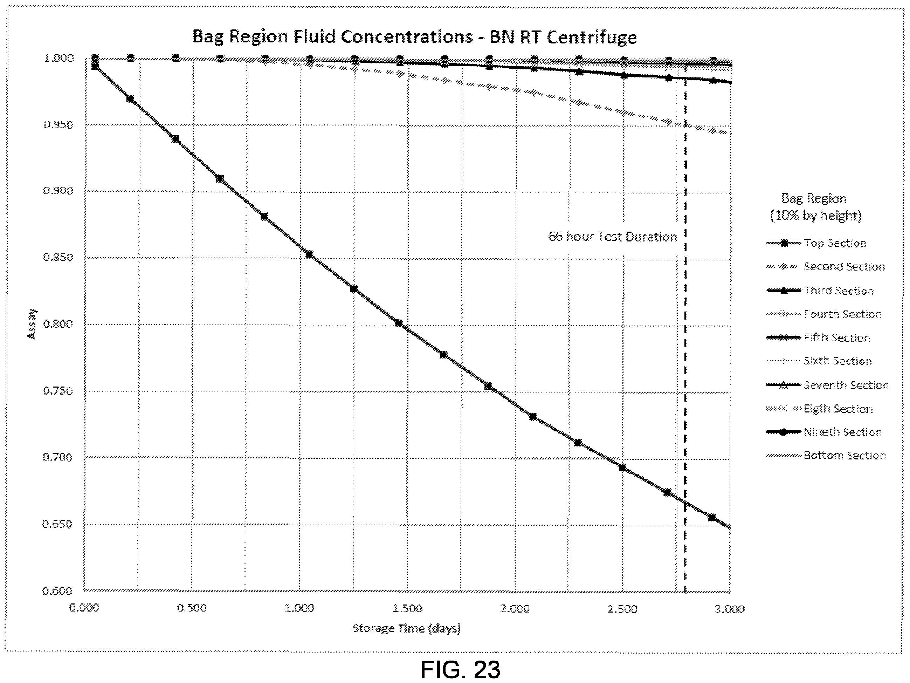

The drug delivery reservoirs were mounted at a 3 foot radius to reduce or minimize differences in acceleration within the drug delivery reservoir. As a result, a varied concentration of the representative formulation throughout the reservoir was produced, as shown for example in FIG. 23. The concentration of the representative formulation after being accelerated for 66 hours is illustrated along the vertical broken line. Each section of the fluid reservoir in the diagram represents one-tenth of the volume of the reservoir from a top section of the reservoir to a bottom section of the reservoir. As shown in FIG. 23, after being accelerated for 66 hours, the top section of the fluid reservoir has about 65-70% of the concentration of BN compared to the bottom section.

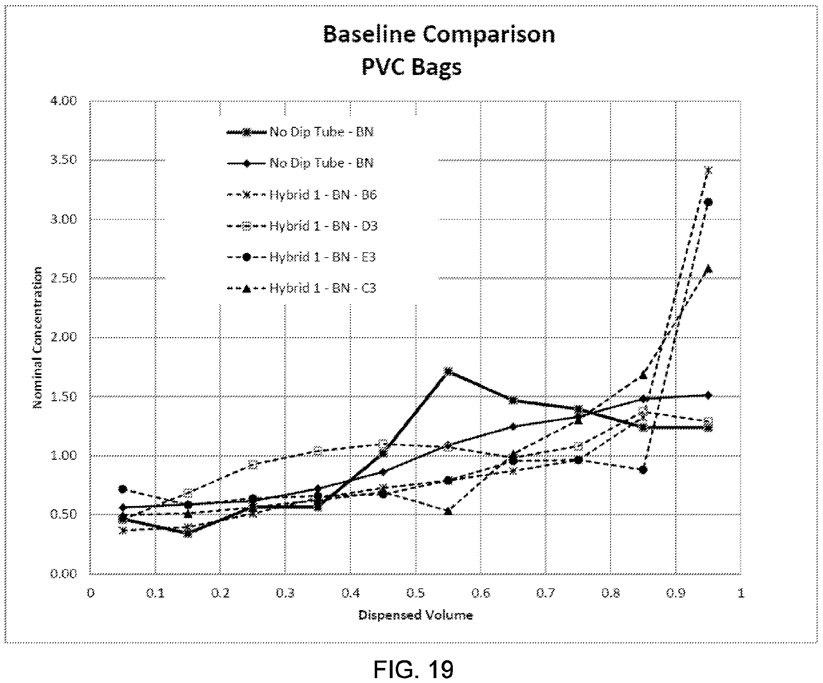

For purpose of comparison with and confirmation of the disclosed subject matter, FIG. 19 is a diagram illustrating exemplary nominal concentration per dispensed volume for drug delivery reservoirs using a dip tube having a constant hole size and spacing (referred to herein as "Hybrid 1" or "Baseline") compared to drug delivery reservoirs using no dip tube. The representative formulation with varied concentration as discussed above with respect to FIG. 23 was utilized, and the fluid was dispensed from the reservoir at a rate of 1 mL/min. With reference to FIG. 19, the drug delivery reservoir using a dip tube having constant hole spacing can draw from the top of the bag initially, and then progressively down into the bag as the bag empties and collapses. For bags without dip tubes, fluid draw can be a function of the bag collapse pattern. The results of the concentration dispensed over the volume for the Hybrid 1 establish a baseline for purpose of comparison with modified aperture configurations discussed herein.

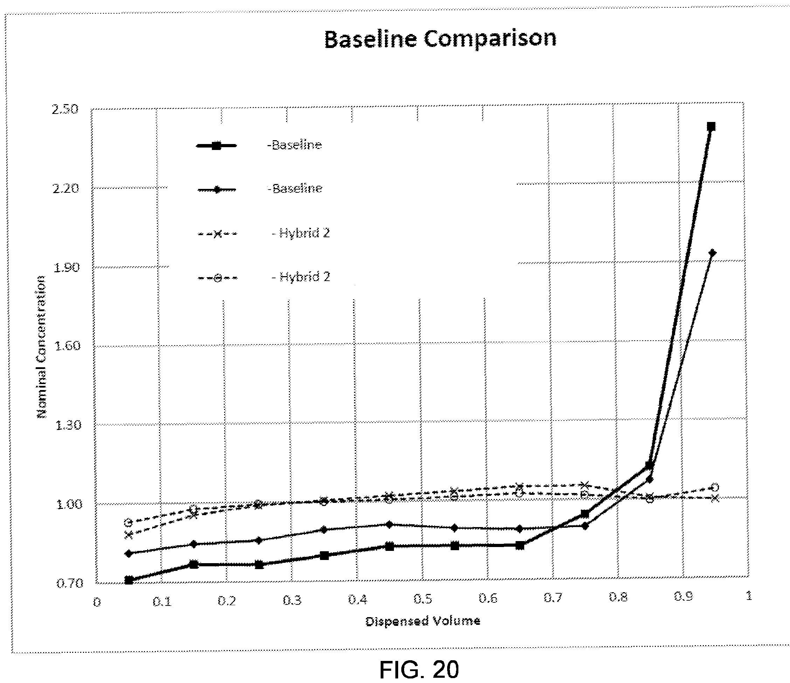

For purpose of comparison and confirmation of the disclosed subject matter, FIG. 20 is a diagram illustrating exemplary nominal concentration per dispensed volume for cassettes using a dip tube having a uniform hole size and spacing (Baseline) compared to cassettes using a dip tube having an aperture configuration described in Table 2 (Hybrid 2). The representative formulation with varied concentration as discussed above with respect to FIG. 23 was utilized, and the fluid was dispensed from the reservoir at a rate of 1 mL/min. As shown in FIG. 20, the aperture configuration of Table 2 provides a relatively consistent fluid concentration of the representative formulation over the entire dispensing volume compared to the Baseline.

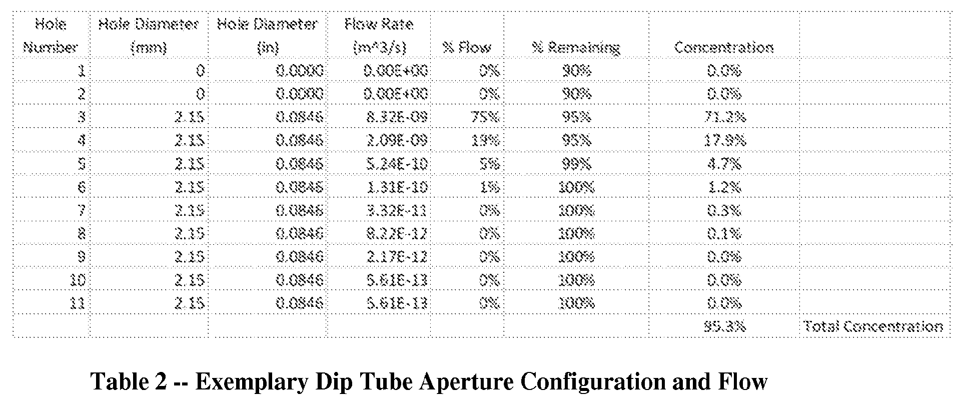

Table 3 illustrates another exemplary dip tube aperture configuration. Compared to the configuration of Table 1, an additional aperture is added toward the distal end of the dip tube 13, opposite the outlet end 33. For purpose of comparison and confirmation of the disclosed subject matter, using a known dip tube with constant aperture sizes and uniform spacing, about 95% of fluid flowed into the dip tube 13 from the first two hole locations during a flow period. The % flow indicates a percentage of fluid taken into the dip tube 13 through the aperture or apertures 14 formed at the corresponding hole location during the flow period. For purpose of comparison and confirmation of the disclosed subject matter, as illustrated in Table 2, flow uniformity is improved over dip tube configurations having constant diameter apertures and uniform spacing. As such, when used with a product having variable concentration, the increased flow uniformity can reduce variations in concentration by drawing fluid at different rates from different locations.

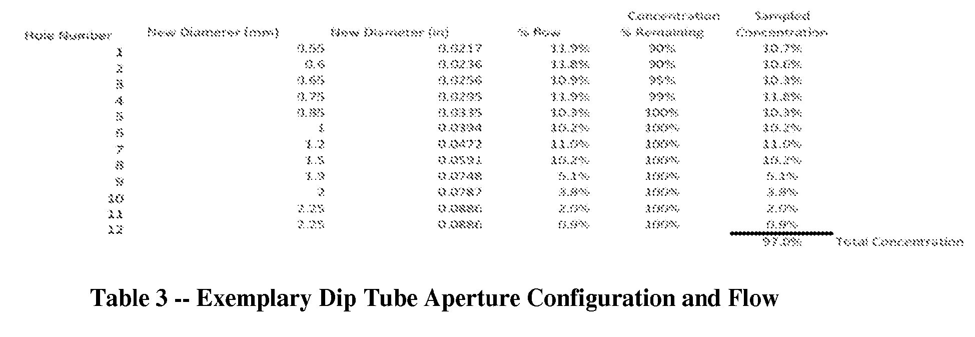

Table 4 illustrates another exemplary dip tube aperture configuration. As shown, relatively smaller apertures were formed in the first 3 hole locations, and larger apertures were formed in 9 subsequent hole locations. For purpose of comparison and confirmation of the disclosed subject matter, as illustrated in Table 4, flow uniformity is improved for the representative formulation over dip tube configurations having constant diameter apertures and uniform spacing.

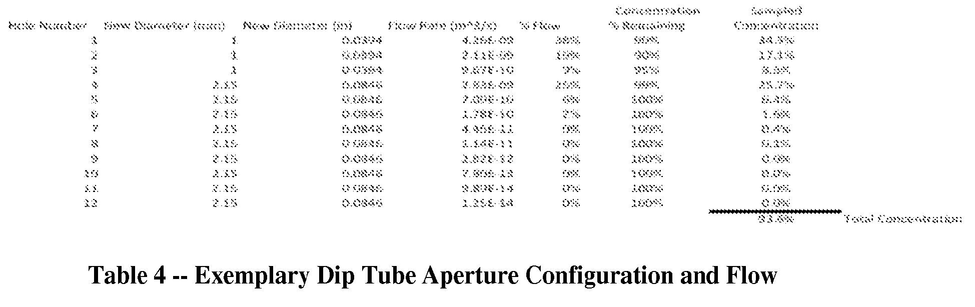

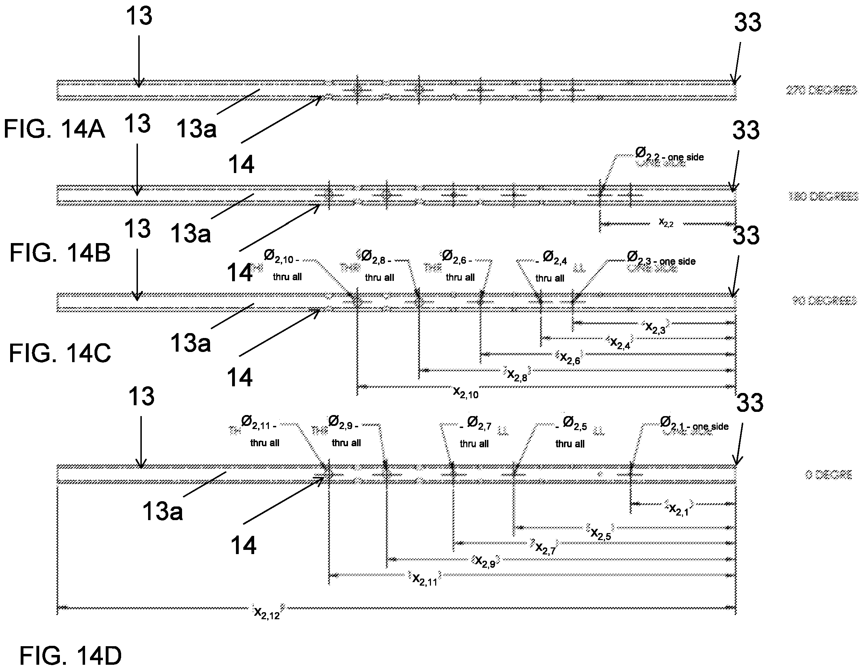

Table 5-1 illustrates another exemplary dip tube aperture configuration. As shown, no aperture was formed in hole location 1, and a non-uniform aperture spacing is used. In hole positions 2.0, 2.9, 3.8 and 4.8, a single hole is formed in the dip tube at the corresponding axial distance. In the subsequent hole positions, two holes were formed in the dip tube at the corresponding axial distance, for example, by forming a through-hole. Table 5-2 and FIG. 14 illustrates another exemplary dip tube aperture configuration. As shown, no aperture was formed in hole location 1, and a non-uniform aperture spacing is used. In hole positions 2.0, 2.9 and 3.8, a single hole is formed in the dip tube at the corresponding axial distance. In subsequent hole positions, two holes were formed in the dip tube at the corresponding axial distance, for example, by forming a through-hole. For purpose of comparison and confirmation of the disclosed subject matter, as illustrated in Tables 5-1 and 5-2, flow uniformity is improved for the representative formulation over dip tube configurations having constant diameter apertures, uniform spacing, and apertures formed closer to the outlet end 33 of the dip tube 13.

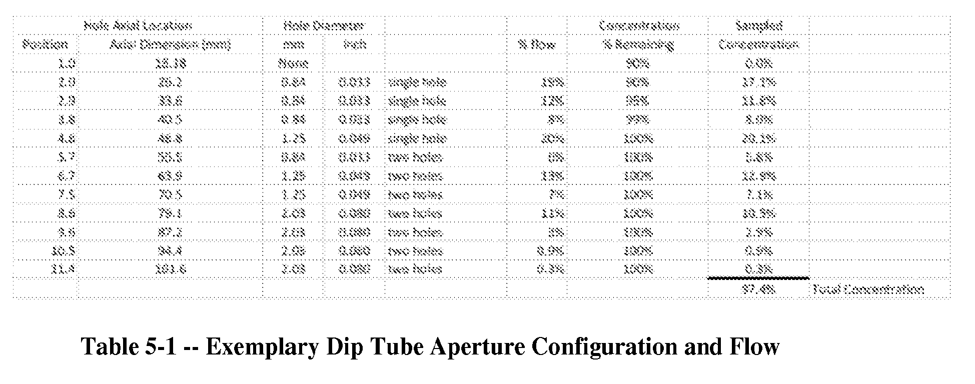

**Table 5-2 -- Exemplary Dip Tube Aperture Configuration and Flow**

| Hole Axial Location | | Hole Diameter | | | Flown Rate | | |
|---|---|---|---|---|---|---|---|
| Position | Axial Dimension (mm) | mm | inch | | m^3/s | ml/hr | % flow |
| 1 | 18.18 | None | | | | | |
| 2 | 26.2 (x_{2,1}) | 0.84 (*φ*_{2,1}) | 0.033 | single hole | 2.22E-09 | 8.00E+00 | 20% |
| 2.9 | 33.6 (x_{2,2}) | 0.84(*φ*_{2,2}) | 0.033 | single hole | 1.48E-09 | 5.33E+00 | 13% |
| 3.8 | 40.5 (x_{2,3}) | 0.84(*φ*_{2,3}) | 0.033 | single hole | 9.99E-10 | 3.60E+00 | 9% |
| 4.8 | 48.4 (x_{2,4}) | 0.84 (*φ*_{2,4}) | 0.033 | two holes | 1.23E-09 | 4.42E+00 | 11% |
| 5.6 | 55.1 (x_{2,5}) | 0.84(*φ*_{2,5}) | 0.033 | two holes | 7.58E-10 | 2.73E+00 | 7% |
| 6.7 | 63.5 (x_{2,6}) | 1.25 (*φ*_{2,6}) | 0.049 | two holes | 1.64E-09 | 5.92E+00 | 15% |
| 7.5 | 70.1 (x_{2,7}) | 1.25 (*φ*_{2,7}) | 0.049 | two holes | 9.07E-10 | 3.26E+00 | 8% |
| 8.6 | 78.7 (x_{2,8}) | 2.03 (*φ*_{2,8}) | 0.08 | two holes | 1.34E-09 | 4.84E+00 | 12% |
| 9.6 | 86.8 (x_{2,9}) | 2.03(*φ*_{2,9}) | 0.08 | two holes | 3.72E-10 | 1.34E+00 | 3% |
| 10.5 | 94(x_{2,10}) | 2.03 (*φ*_{2,10}) | 0.08 | two holes | 1.14E-10 | 4.09E-01 | 1% |
| 11.4 | 101.2 (x_{2,11}) | 2.03 (*φ*_{2,11}) | 0.08 | two holes | 4.30E-11 | 1.55E-01 | 0% |
| Total length | 168.69 (x_{2,12}) | | | | | 4.00E+01 | |

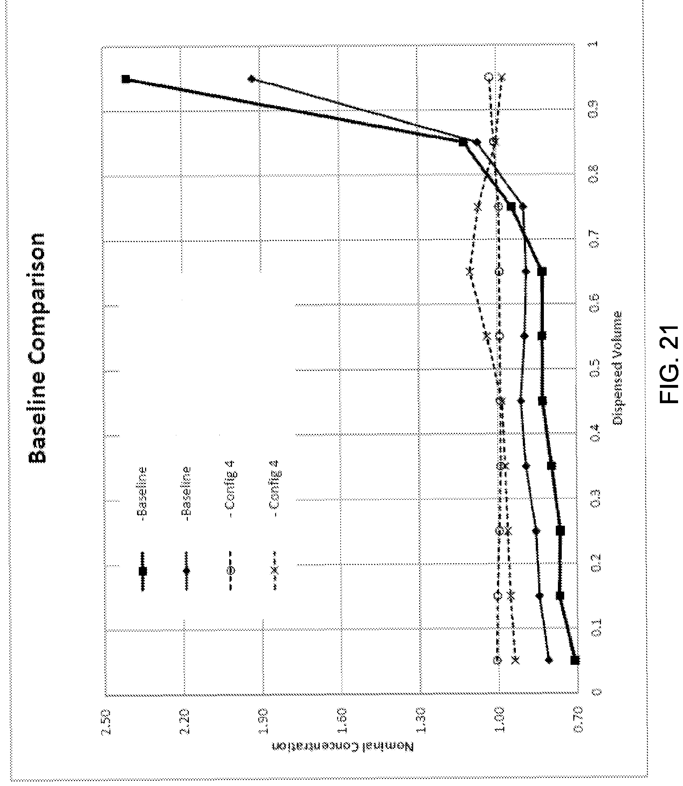

Table 6 illustrates the exemplary dip tube aperture configuration of FIGS. 13A-13C. As shown, no aperture was formed in hole location 1, and a non-uniform aperture spacing is used. For purpose of comparison and confirmation of the disclosed subject matter, as illustrated in Table 6, flow uniformity is improved over known dip tube configurations having constant diameter apertures, uniform spacing, and apertures formed closer to the outlet end 33 of the dip tube 13. FIG. 21 is a diagram illustrating exemplary nominal concentration per dispensed volume for drug delivery reservoirs using a dip tube having a uniform hole size and spacing (Baseline) compared to drug delivery reservoirs using a dip tube having an aperture configuration described in Table 6 (Config 4). The representative formulation with varied concentration as discussed above with respect to FIG. 23 was utilized, and the fluid was dispensed from the reservoir at a rate of 1 mL/min. As shown, using the aperture configuration of Table 6, the dispensed concentration was substantially uniform over the entire displacement from the bag, and thus provides about a nine-fold improvement in dose accuracy compared to the uniform hole dip tube for the representative formulation.

**Table 6 -- Exemplary Dip Tube Aperture Configuration and Flow**

| Hole Axial Location | | Hole Diameter | | | | Concentration | Sampled |
|---|---|---|---|---|---|---|---|
| Position | Axial Dimension (mm) | mm | inch | | % flow | % Remaining | Concentration |
| 1 | 18.18 | None | | | | 90% | 0.00% |
| 2 | 26.2 (x_{1,1}) | 0.51 (*φ*_{1,1}) | 0.02 | four holes | 13% | 90% | 11.30% |
| 2.9 | 33.6 (x_{1,2} | 0.51 (*φ*_{1,2}) | 0.02 | four holes | 8% | 95% | 7.80% |
| 3.8 | 40.5 (x_{1,3}) | 0.84(*φ*_{1,3}) | 0.033 | two holes | 19% | 99% | 18.40% |
| 4.8 | 48.4 (x_{1,4}) | 0.84(*φ*_{1,4}) | 0.033 | two holes | 11% | 100% | 11.20% |
| 5.6 | 55.1 (x_{1,5}) | 0.84 (*φ*_{1,5}) | 0.033 | three holes | 10% | 100% | 10.10% |
| 6.7 | 63.5 (x_{1,6}) | 1.24 (*φ*_{1,6}) | 0.049 | two holes | 15% | 100% | 14.60% |
| 7.5 | 70.1(x_{1,7}) | 1.24 (*φ*_{1,7}) | 0.049 | two holes | 8% | 100% | 8.10% |
| 8.6 | 78.7 (x_{1,8}) | 2.03 (*φ*_{1,8}) | 0.08 | two holes | 12% | 100% | 12.00% |
| 9.6 | 86.8 (x_{1,9}) | 2.03 (*φ*_{1,9}) | 0.08 | two holes | 3% | 100% | 3.30% |
| 10.5 | 94(x_{1,10}) | 1.65 (*φ*_{1,10}) | 0.065 | four holes | 1% | 100% | 1.00% |
| 11.4 | 101.2 (x_{1,11}) | 1.65 (*φ*_{1,11}) | 0.065 | four holes | 0% | 100% | 0.40% |
| Total length | 168.69 (x_{1,12}) | | | | | | 98.10% |

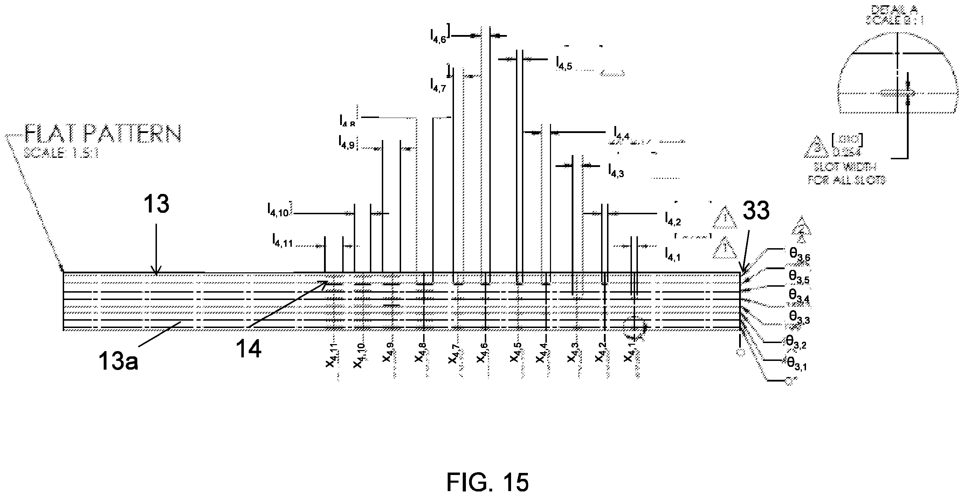

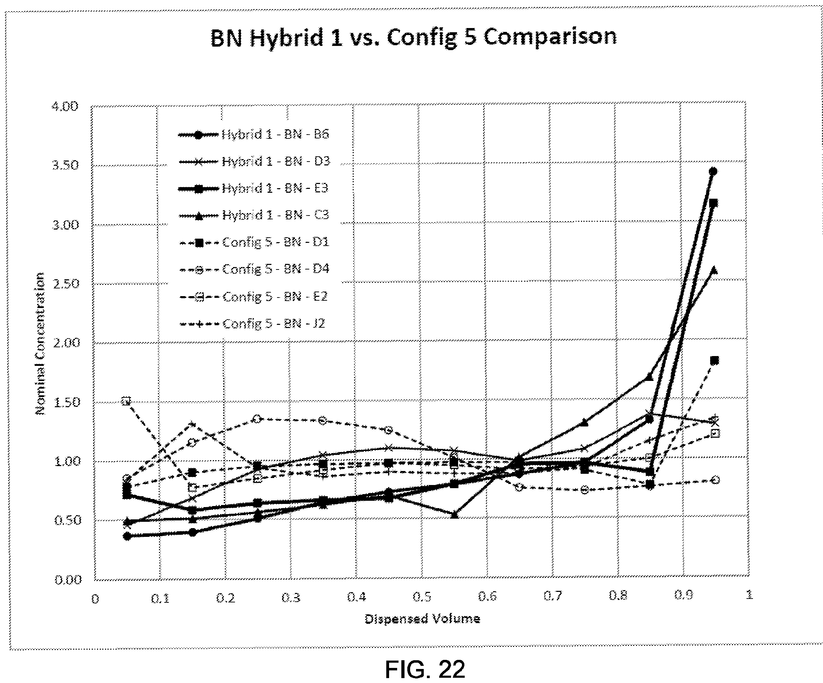

Table 7 and FIG. 15 illustrate another exemplary dip tube aperture configuration. FIG. 15 shows the exemplary dip tube 13 in a flattened configuration, for purpose of illustration of the configuration of apertures 14 of the dip tube 13. In the configuration of Table 7 and FIG. 15, a slotted aperture configuration is used. Additionally, no aperture is formed in hole location 1, and a non-uniform slot length is used. For purpose of comparison and confirmation of the disclosed subject matter, as illustrated in Table 7, flow uniformity is improved over known dip tube configurations having constant diameter apertures, uniform spacing, and apertures formed closer to the outlet end 33 of the dip tube 13. FIG. 22 is a diagram illustrating exemplary nominal concentration per dispensed volume for cassettes using a dip tube having a uniform hole size and spacing (Hybrid 1) compared to drug delivery reservoirs using a dip tube having an aperture configuration described in Table 7 (Config 5). The representative formulation with varied concentration as discussed above with respect to FIG. 23 was utilized, and the fluid was dispensed from the reservoir at a rate of 1 mL/min. As shown, the configuration of Table 7 provides about a 50% improvement in dose accuracy versus the uniform hole dip tube for the representative formulation. Additionally, the configuration of Table 7 can reduce or minimize the effects of manufacturing tolerances by providing a substantially uniform slit width.

**Table 7 -- Exemplary Dip Tube Aperture Configuration and Flow (Slotted)**

| | | Total Area | higth = Are a/0.01" | no:of slots | length per slot | Flow Rate | | | Concentration | Sampled |
|---|---|---|---|---|---|---|---|---|---|---|
| Location | Axial Dimension (mm) inch^2 | | inch: | | inch | m ^3/s | ml/hr | flow | % Remaining | Concentration |
| 1 | None | | | | | | | | 90% | 0.00% |
| 2 | 26.2 (x_{4,1}) | 0.001 | 0.126 | 2 | 0.0628 (I_{4,1}) | 1.39E-09 | 5.01E+00 | 13% | 90% | 11.30% |
| 3 | 33.6 (x_{4,2}) | 0.001 | 0.126 | 2 | 0.0628 (I_{4,2}) | 1.10E-09 | 3.98E+00 | 10% | 95% | 9.50% |
| 4 | 40.5 (x_{4,3}) | 0.002 | 0.171 | 2 | 0.0855 (I_{4,3}) | 1.26E-09 | 4.55E+00 | 11% | 99% | 11.30% |
| 5 | 48.4 (x_{4,4}) | 0.002 | 0.171 | 2 | 0.0855 (I_{4,4}) | 9.09E-10 | 3. 27E+00 | 8% | 100% | 8.20% |
| 6 | 55.1 (x_{4,5}) | 0.003 | 0.257 | | 0.0641 (I_{4,5}) | 9.09E-10 | 3.27E+00 | 8% | 100% | 8.20% |
| 7 | 63.5 (x_{4,6}) | 0.004 | 0.377 | 4 | 0.0943 (I_{4,6}) | 9.75E-10 | 3.51E+00 | 9% | 100% | 8.80% |
| 8 | 70.1 (x_{4,7}) | 0.004 | 0.377 | 4 | 0.0943 (I_{4,7}) | 7.11E-10 | 2.56E+00 | 6% | 100% | 6.40% |
| 9 | 78.7 (x_{4,8}) | 0.01 | 1.005 | 6 | 0.1676 (I_{4,8}) | 1.31E-09 | 4.72E+00 | 12% | 100% | 11.80% |
| 10 | 86.8 (x_{4,9}) | 0.01 | 1.005 | 6 | 0.1676 (I_{4,9}) | 9.13E-10 | 3.29E+00 | 8% | 100% | 8.20% |
| 11 | 94 (x_{4,10}) | 0.013 | 1.327 | 8 | 0.1659 (I_{4,10}) | 8.77E-10 | 3.16E+00; | 8% | 100% | 7.90% |
| 12 | 101.2 (x_{4,11}) | 0.013 | 1.327 | 8 | 0.1659 (I_{4,11}) | 7.29E-10 | 2.63E+00 | 7% | 100% | 6.60% |
| | | | | | | | 399E+01 | | | 98.10% |

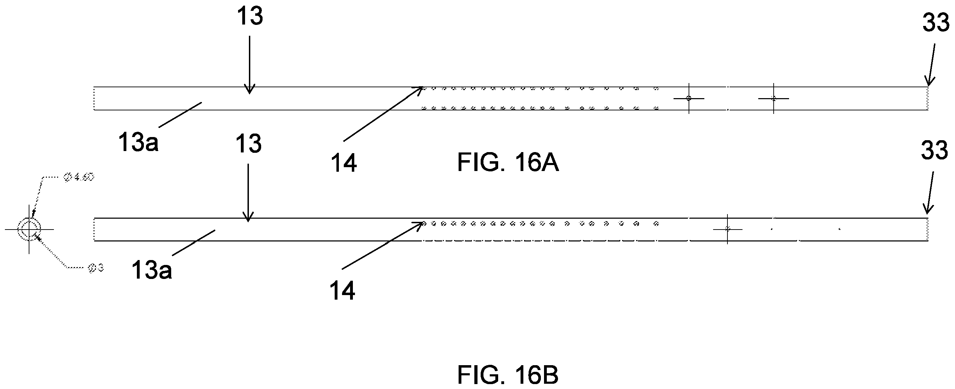

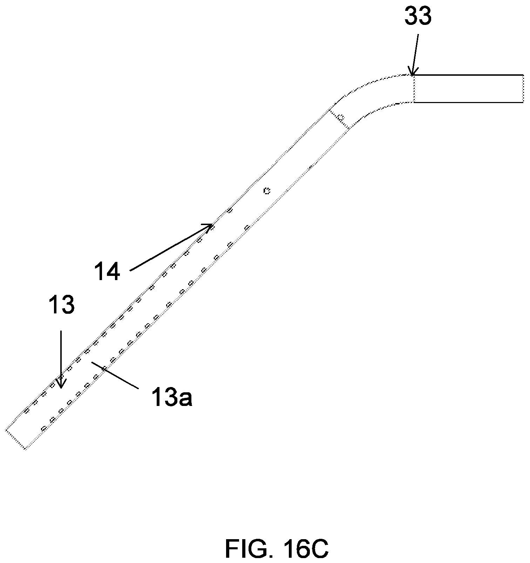

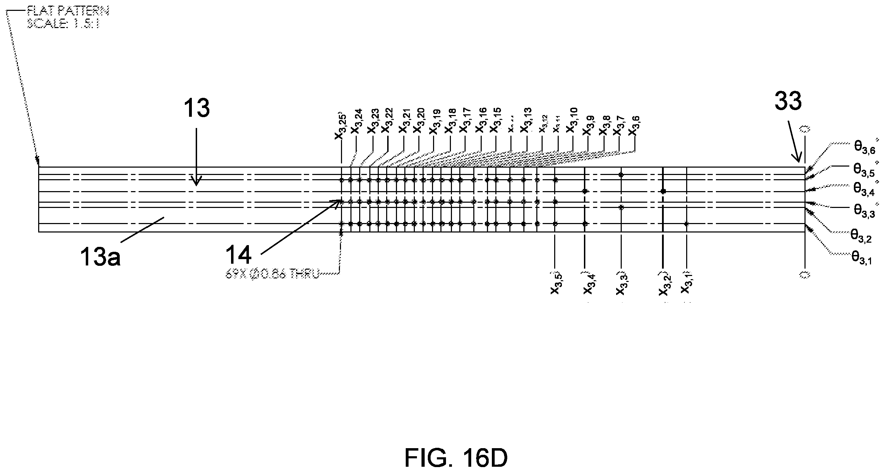

Table 8 and FIG. 16A illustrate another exemplary dip tube aperture configuration. FIG. 16B shows the exemplary dip tube 13 rotated 90 degrees with respect to FIG. 16A. FIG. 16C shows the exemplary dip tube 13 joined to an exemplary peristaltic tube. FIG. 16D shows the exemplary dip tube 13 in a flattened configuration, for purpose of illustration of the configuration of apertures 14 of the dip tube 13. In the configuration of Table 8, as embodied herein, each aperture has the same diameter. Additionally, no aperture is formed in hole location 1. Flow distribution can be adjusted by the number of holes at each location and the spacing between holes. For purpose of comparison and confirmation of the disclosed subject matter, as illustrated in Table 8, flow uniformity is improved for the representative formulation over known dip tube configurations having constant diameter apertures, uniform spacing, and apertures formed closer to the outlet end 33 of the dip tube 13.

**Table 8 -- Exemplary Dip Tube Aperture Configuration and Flow**

| Hole Axial | Hole Diameter | | | Flow Rate | | Concentration | Sampled |
|---|---|---|---|---|---|---|---|
| Dimension | mm | inch | No: of holes | m^3/2 | ml/hr | % Remaining ' | Concentration |
| 26.2 (x_{3,1}) | 0.86 | 0.0339 | 1(θ_{3,1}) | 2.04E-09 | 7.33E+00 | 90 % | 16.50% |
| 31.2 (x_{3,2}) | 0.86 | 0.0339 | 1(θ_{3,4}) | 1.53E-09 | 5.51E+00 | 95% | 13.10% |
| 40.5 (x_{3,3}) | 0.86 | 0.0339 | 2(θ_{3,2}, θ_{3,6}) | 1.75E-09 | 6.29E+00 | 99% | 15.60% |
| 48.4 (x_{3,4}) | 0.86 | 0.0339 | 2(θ_{3,1}, θ_{3,4}) | 1.05E-09 | 3.79E+00 | 100% | 9.50% |
| 55 (x_{3,5}) | 0.86 | 0.0339 | 3(θ_{3,1}, θ_{3,3}, θ_{3,5}) | 9.83E-10 | 3.54E+00 | 100% | 8.80% |
| 59 (x_{3,6}) | 0.86 | 0.0339 | 3(θ_{3,1}, θ_{3,3}, θ_{3,5}) | 7.34E-10 | 2.64E+00 | 100% | 6.60% |
| 62 (x_{3,7}) | 0.86 | 0.0339 | 3(θ_{3,1}, θ_{3,3}, θ_{3,5}) | 5.84E-10 | 2.10E+00 | 100% | 5.30% |
| 65 (x_{3,8}) | 0.86 | 0.0339 | 3(θ_{3,1}, θ_{3,3}, θ_{3,5}) | 4.61E-10 | 1.66E+00 | 100% | 4.10% |
| 68 (x_{3,9}) | 0.86 | 0.0339 | 3(θ_{3,1}, θ_{3,3}, θ_{3,5}) | 3.62E-10 | 1.30E+00 | 100% | 3.30% |
| 70 (x_{3,10}) | 0.86 | 0.0339 | 3(θ_{3,1}, θ_{3,3}, θ_{3,5}) | 3.07E-10 | 1.11E+00 | 100% | 2.80% |
| 73 (x_{3,11}) | 0.86 | 0.0339 | 3(θ_{3,1}, θ_{3,3}, θ_{3,5}) | 2.40E-10 | 8.64E-01 | 100% | 2.20% |
| 76 (x_{3,12}) | 0.86 | 0.0339 | 3(θ_{3,1},θ_{3,3},θ_{3,5}) | 1.86E-10 | 6.68E-01 | 100% | 1.70% |
| 78 (x_{3,13}) | 0.86 | 0.0339 | 3(θ_{3,1}, θ_{3,3}, θ_{3,5}) | 1.55E-10 | 5.56E-01 | 100% | 1.40% |
| 80 (x_{3,14}) | 0.86 | 0.0339 | 3(θ_{3,1}, θ_{3,3}, θ_{3,5}) | 1.29E-10 | 4.64E-01 | 100% | 1.20% |
| 82 (x_{3,15}) ' | 0.86 | 0.0339 | 3(θ_{3,1}, θ_{3,3}, θ_{3,5}) | 1.08E-10 | 3.88E-01 | 100% | 1.00% |
| 84 (x_{3,16}) | 0.86 | 0.0339 | 3(θ_{3,1}, θ_{3,3}, θ_{3,5}) | 9.01E-11 | 3.24E-01 | 100% | 0.80% |
| 86 (x_{3,17}) | 0.86 | 0.0339 | 3(θ_{3,1}, θ_{3,3}, θ_{3,5}) | 7.57E-11 | 2.72E-01 | 100% | 0.70% |
| 88 (x_{3,18}) | 0.86 | 0.0339 | 3(θ_{3,1}, θ_{3,3}, θ_{3,5}) | 6.37E-11 | 2.29E-01 | 100% | 0.60% |
| 90 (x_{3,19}) | 0.86 | 0.0339 | 3(θ_{3,1}, θ_{3,3}, θ_{3,5}) | 5.40E-11 | 1.94E-01 | 100% | 0.50% |
| 92 (x_{3,20}) | 0.86 | 0.0339 | 3(θ_{3,1}, θ_{3,3}, θ_{3,5}) | 4.64E-11 | 1.67E-01, | 100% | 0.40% |
| 94 (x_{3,21}) ' | 0.86 | 0.0339 | 3(θ_{3,1}, θ_{3,3}, θ_{3,5}) | 4.02E-11 | 1.45E-01 | 100% | 0.40% |
| 96 (x_{3,22}) ' | 0.86 | 0.0339 | 3(θ_{3,1}, θ_{3,3}, θ_{3,5}) | 3.56E-11 | 1.28E-01 | 100% | 0.30% |
| 98 (x_{3,23}) | 0.86 | 0.0339 | 3(θ_{3,1}, θ_{3,3}, θ_{3,5}) | 3.23E-11 | 1.16E-01 | 100% | 0.30% |
| 100 (_{X3,24}) | 0.86 | 0.0339 | 3(θ_{3,1}, θ_{3,3}, θ_{3,5}) | 3.01E-11 | 1.08E-01 | 100% | 0.30% |
| 102 (x_{3,25}) | 0.86 | 0.0339 | 3(θ_{3,1}, θ_{3,3}, θ_{3,5}) | 2.90E-11 | 1.04E-01 | 100% | 0.30% |
| | | | | | 4.00E+01 | | 97.30% |

As such, and as demonstrated above, the plurality of apertures can be configured to provide a generally uniform distribution of flow through the plurality of apertures along the length of the tubular member. For purpose of illustration and not limitation, and as embodied herein, the plurality of apertures can vary in spacing between adjacent apertures along the length of the tubular wall. For example, and as embodied herein, the plurality of apertures can decrease in spacing toward the distal end of the tubular wall. Additionally, in combination with any or all of the above configurations, or alternatively, the plurality of apertures can vary in cross dimension along the length of the tubular wall. For example, and as embodied herein, the plurality of apertures can increase in cross dimension along the length of the tubular wall. Furthermore, in combination with any or all of the above configurations, or as a further alternative, the plurality of apertures can have one or more shapes, for example and without limitation, a slotted shape, a circular shape, and/or any other suitable shape. In addition, in combination with any or all of the above configurations, or as another alternative, one of the plurality of apertures nearest the outlet end can be spaced from the outlet end a distance of at least 15% of the length of the tubular wall, and as embodied herein, the one of the plurality of apertures nearest the outlet end can be spaced from the outlet a distance of about 20% of the length of the tubular wall. Moreover, in combination with any or all of the above configurations, or as another alternative, at least two of the plurality of apertures can be aligned axially along the length of the tubular wall and spaced circumferentially about the tubular wall. As described herein, in accordance with the disclosed subject matter, a fluid beneficial agent in the reservoir can have a volume and a concentration increasing from a region proximate the outlet end to a region proximate the distal end, and as embodied herein, the dip tube can be configured to deliver the volume of the fluid beneficial agent at a substantially uniform concentration.

Furthermore, and as embodied herein, flow accuracy of the peristaltic pump can be improved by controlling the tension of the peristaltic tube 23. As embodied herein, the tube tension fit can be achieved by controlling the length and diameter of the peristaltic tube 23 to achieve a desired tension. That is, reducing the length of the peristaltic tube 23 increases tension and reduces the overall flow rate. Increasing the length of the peristaltic tube 23 reduces tension and can cause buckling in the peristaltic tube 23 and create issues with installation and repeatability.

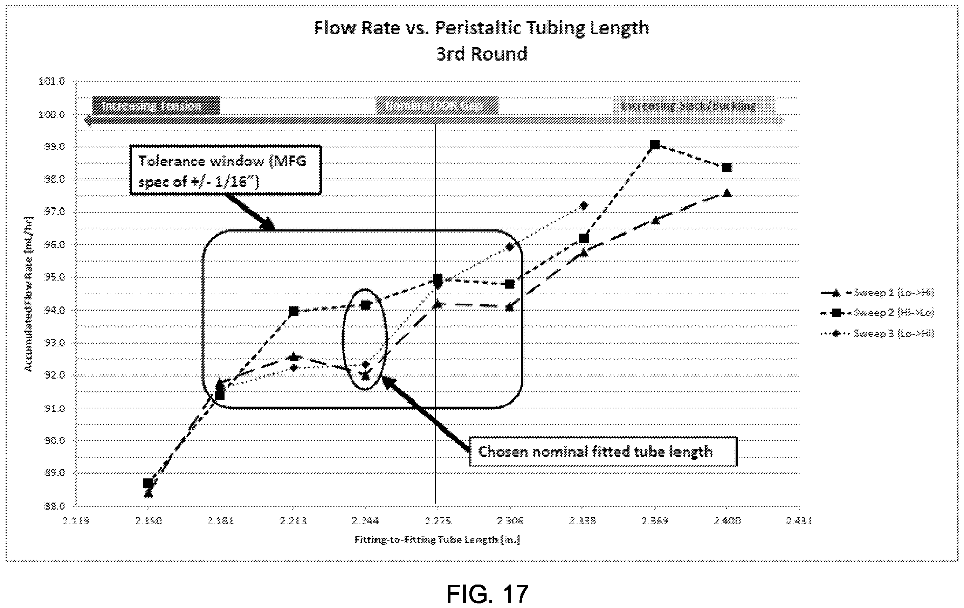

FIG. 17 is a diagram illustrating the accumulated flow rate (mL/hr) plotted against fitting-to fitting length (in.) for a section of peristaltic tubing with a nominal length of 2.275" stretched or relaxed by +/1 1/8" in 1/32" steps. The tests were run with 3 samples of each tubing length, sweeping test runs from low length to high length, then back from high to low, to randomize runs and allow for data on all lengths. FIG. 16 shows that as tension was increased, the flow rate decreased. In contrast, as slacking/buckling increased, flow rate increased. Furthermore, FIG. 17 illustrates that for a peristaltic tube having a nominal length of 2.275" and stretched about a 1/32" there is a tolerance window of +/- 1/16" such that the flow rate will remain between about 91 ml/hr and 96 ml/hr.

For example, and as embodied herein, peristaltic tube 23 can be stretched at least about 0.782 mm (0.031 in). The specific length can be held in place by the cassette housing 11, along with elbow fitting 16 and junction fitting 24. Controlling the tension of the peristaltic tube 23 can allow for increased pump flow accuracy and repeatability.

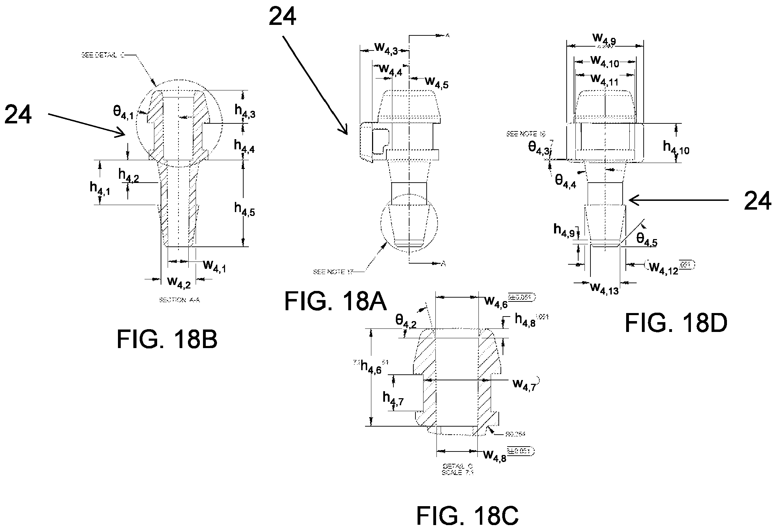

FIG. 18 illustrates an exemplary junction fitting 24. A tubing clamp 25 (illustrated in FIG. 5B) can be connected to the delivery tube 20 to allow a user to align and secure the tubing at a desired orientation and position. Additionally, as embodied herein, a connection sub-assembly 26 (illustrated in FIG. 5B) can be provided on the end of delivery tube 20 to allow the tubing and reservoir to be joined to a pump device. Representative dimensions of an exemplary junction fitting, for purpose of illustration and not limitation, are set forth below.

| **FIG. 18** | **Exemplary Dimensions (mm)** |
|---|---|
| w_{4,1} | 2.378 |
| w_{4,2} | 4.051 |
| w_{4,3} | 5.692 |
| w_{4,4} | 4.241 |
| w_{4,5} | 1.944 |
| w_{4,6} | 3.556±0.051 |
| w_{4,7} | 5.56 |
| w_{4,8} | 3.378±0.051 |
| w_{4,9} | 8.94 |
| w_{4,10} | 7.2 |
| w_{4,11} | 6.85 |
| w_{4,12} | 4.775±0.051 |
| w_{4,13} | 3.378 |
| h_{4,1} | 5.082 |
| h_{4,2} | 2.542 |
| h_{4,3} | 3.75 |
| h_{4,4} | 4.2 |
| h_{4,5} | 9.855 |
| h_{4,6} h_{4,6} | 7.950±0.051 |
| h_{4,7} | 3 |
| h_{4,8} | 0.787±0.051 |
| h_{4,9} | 0.508 |
| h_{4,10} | 4.5 |

| **FIG. 18** | **Examplary Dimensions (deg)** |
|---|---|
| θ_{4,1} | 13.1° |
| θ_{4,2} | 74° |
| θ_{4,3} | 1.0° |
| θ_{4,4} | 9.6° |
| θ_{4,5} | 45.000° |

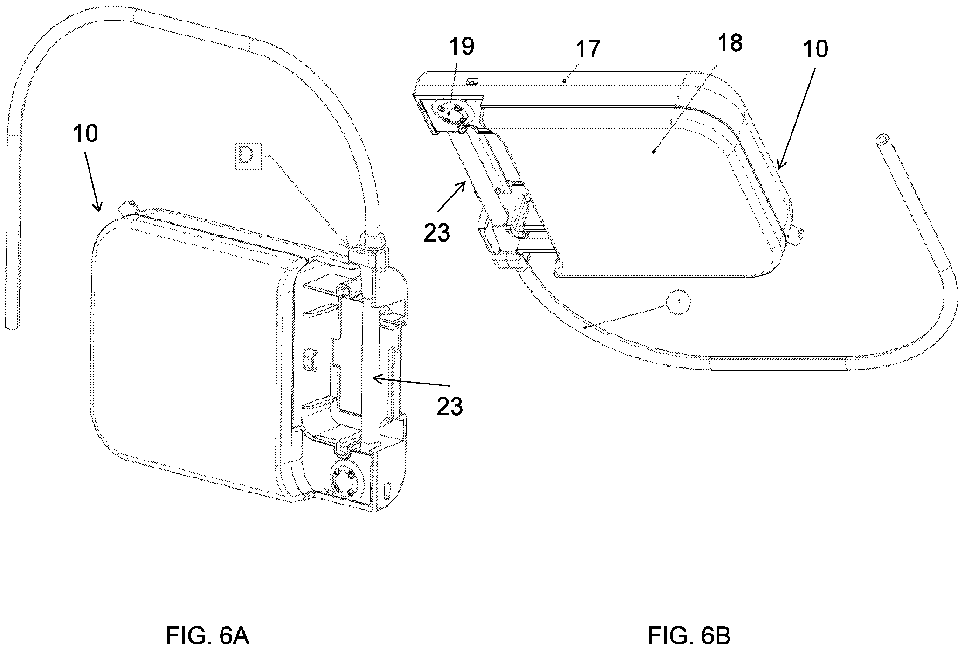

If formed separately, the fluid reservoir 12 can be installed into the cassette housing 11. For example, and as embodied herein, the cassette housing 11 can be configured with two enclosure clamshell portions 17 and 18 (as shown for example in FIG. 6B), which can receive and contain the fluid reservoir 12. The two clamshell portions 17 and 18 can be adhered or otherwise joined together, for example by ultrasonic welding. Furthermore, and as embodied herein, the peristaltic tube portion 23 can be received by a RFID enclosure shell 19 on one side of the cassette housing 11 and by a frictional engagement (for example as denoted by "D" in FIG. 6A) or other receiving feature on an opposing side of the cassette housing 11. As such, the peristaltic tube 23 can be suspended within the housing 11, which can allow for increased shape or dimensional control of the peristaltic tube 23 inside the pump mechanism, and can reduce the profile of the peristaltic tube 23 within the cassette housing 11.

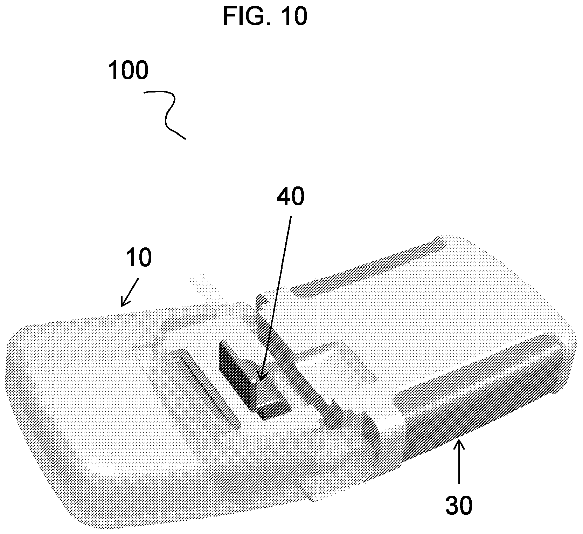

As previously noted, the cassette 10 disclosed herein can be used with a variety of pumps or similar fluid delivery devices. For purpose of illustration and not limitation, reference is made to the pump 30 of FIGS. 1, 2 and 7-12. The pump 30 can include a pump housing 31. The pump housing 31 can include a pump assembly having a fluid drive component. The pump assembly can be configured, for example, as a peristaltic pump. For example, a peristaltic pump can include, a motor, a cam shaft, and a plurality of finger plates disposed along the length of the cam shaft. The cam shaft can be coupled to the motor for rotation about a longitudinal axis of the cam shaft, and can have at least one radially-outward projection defining a helical engagement portion disposed along a length of the cam shaft. The plurality of finger plates can be disposed along the length of the cam shaft. Each finger plate can be mounted for movement in a transverse direction relative to the longitudinal axis of the cam shaft, and can have an aperture defined therein to receive the cam shaft therethrough.

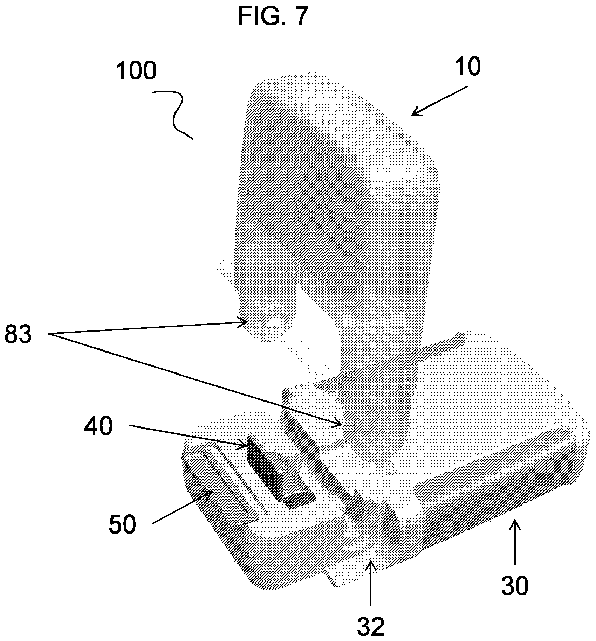

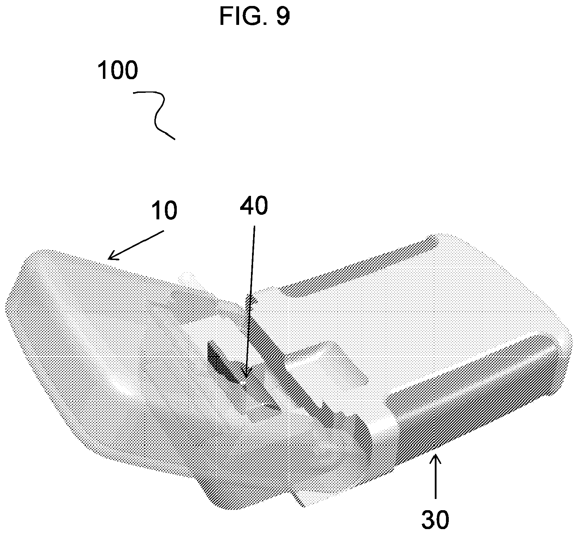

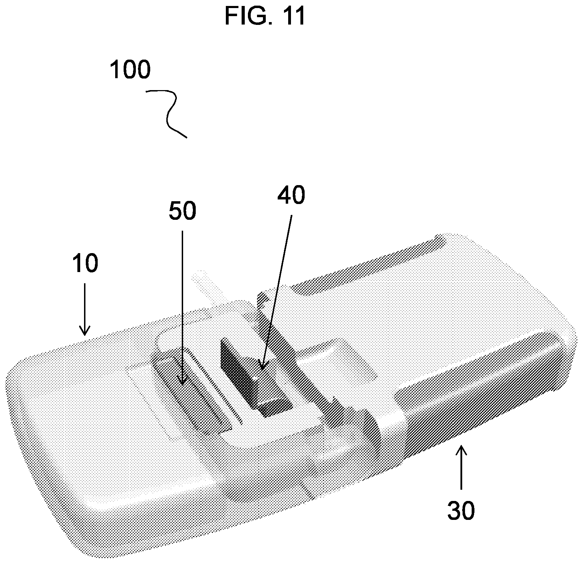

The pump housing 31 can have a receiving region 32 (for example as shown in FIG. 7) to receive the cassette base region 83. The fluid drive component can be disposed proximate the receiving region. For example, and as embodied herein, the cassette 10 can be joined to the receiving region 32 of the pump 30 with the delivery tube or peristaltic tube, if provided, in alignment with the fluid drive component of the pump 30. Furthermore, and as embodied herein, the cassette 10 can be received by the pump housing 31 at a 90 degree angle (as shown for example in FIGS. 7-8) and rotated 90 degrees relative the pump housing 31 into an orientation generally parallel with the pump housing 31 (as shown for example in FIGS. 9-11). The cassette 10 can be rotated into engagement with a spring loaded clip 50 (shown in FIG. 11), to thereby retain the cassette 10 within the housing 31. Furthermore, and as embodied herein, the clip 50 can be retracted to disengage the cassette 10 from the housing 31.

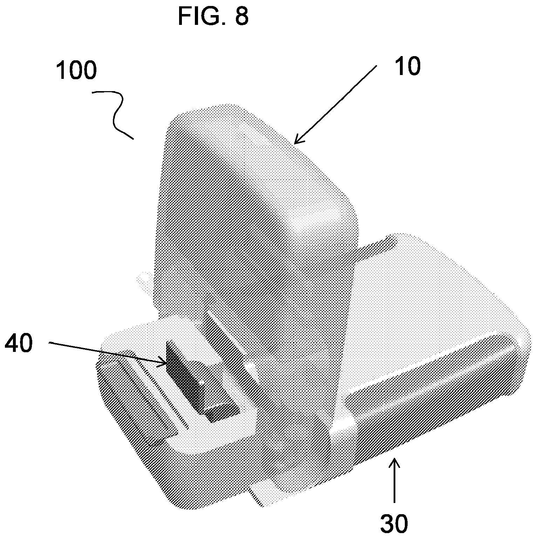

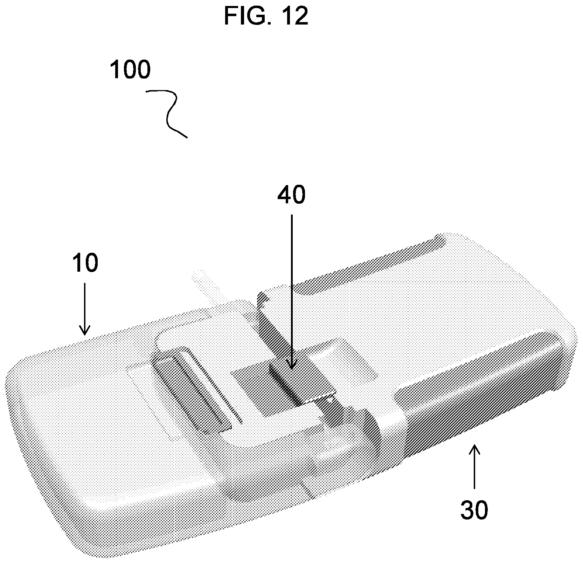

As shown in FIGS. 7-12, for the purpose of illustration and not limitation, the device 100 can include a latch 40 coupled to the pump housing 31 and movable between an open position and a closed position. FIG. 7 shows an exploded view of the cassette 10 and pump 30 separated. FIGS. 8-11 each sequentially shows the cassette 10 and pump being joined, with latch 40 in the open position. FIG. 12 shows the latch 40 moved to the closed position. The cassette 10 can be inserted into and removed from the receiving region 32 when the latch 40 is in the open position. When the latch 40 is in the closed position, the cassette 10 can be secured to the pump 30 with the cassette base region 83 disposed within the receiving region. The delivery tube and/or peristaltic tube, if provided, can be in operative engagement with the fluid drive component along the length of the delivery tube. The latch 40 can be configured, for example and as embodied herein, as a spring latch. The latch 40 can be disposed within a recess of the pump body 31, and as such, when the latch 40 is in the closed position, the latch 40 can be substantially flush with the pump body 31.

Each of the components described herein can be made of any suitable material (e.g., plastic, composites, metal, etc.) and technique for its intended purpose. In addition to the specific embodiments claimed below, the disclosed subject matter is also directed to other embodiments having any other possible combination of the dependent features claimed below and those disclosed above. As such, the particular features disclosed herein can be combined with each other in other manners within the scope of the disclosed subject matter such that the disclosed subject matter should be recognized as also specifically directed to other embodiments having any other possible combinations. Thus, the foregoing description of specific embodiments of the disclosed subject matter has been presented for purposes of illustration and description. It is not intended to be exhaustive or to limit the disclosed subject matter to those embodiments disclosed.

It will be apparent to those skilled in the art that various modifications and variations can be made in the method and system of the disclosed subject matter without departing from the spirit or scope of the disclosed subject matter. Thus, it is intended that the disclosed subject matter include modifications and variations that are within the scope of the appended claims and their equivalents.

## Claims

1. A drug delivery reservoir for delivery of a beneficial agent to a user, comprising:
a drug delivery reservoir housing (11) having a fluid reservoir (12) defined therein, the drug delivery reservoir housing having a drug delivery reservoir base region (83);
a dip tube (13) extending inside the fluid reservoir (12), the dip tube (13) including a tubular wall defining a flow lumen, the tubular wall having at least one aperture (14) defined therein and spaced proximally from a distal end of the tubular wall in fluid communication with the fluid reservoir (12); and
an adaptor disposed external to the drug delivery reservoir housing (11) and coupled to a proximal end of the dip tube (13).

2. The drug delivery reservoir of claim 1, wherein the fluid reservoir is a flexible bag disposed within the housing.

3. The drug delivery reservoir of claim 1, wherein the dip tube (13) is disposed diagonally across an interior region of the fluid reservoir (12), or wherein the dip tube (13) is disposed along a perimeter of the fluid reservoir (12).

4. The drug delivery reservoir of claim 1, wherein the tubular wall has a plurality of apertures (14) spaced apart along a length of the tubular wall.

5. The drug delivery reservoir of claim 4, wherein the apertures (14) are spaced evenly from each other along a length of the tubular wall.

6. The drug delivery reservoir of claim 4, wherein one of the plurality of apertures (14) nearest the outlet end is spaced from the outlet end a distance of at least 15% of the length of the tubular wall, optionally wherein one of the plurality of apertures (14) nearest the outlet end is spaced from the outlet end a distance of about 20% of the length of the tubular wall.

7. The drug delivery reservoir of claim 4, wherein the plurality of apertures (14) are configured to provide a generally uniform distribution of flow through the plurality of apertures along the length of the tubular member.

8. The drug delivery reservoir of claim 7, wherein the plurality of apertures (14) vary in spacing between adjacent apertures along the length of the tubular wall.

9. The drug delivery reservoir of claim 8, wherein the plurality of apertures (14) decrease in spacing toward the distal end of the tubular wall.

10. The drug delivery reservoir of claim 7, wherein the plurality of apertures (14) vary, and optionally increase, in cross dimension along the length of the tubular wall.

11. The drug delivery reservoir of claim 4, wherein a size of the plurality of apertures (14) increases along the tubular wall from the outlet end toward the distal end.

12. The drug delivery reservoir of claim 4, wherein the plurality of apertures (14) have a slotted or circular shape.

13. The drug delivery reservoir of claim 4, wherein at least two, and optionally at least three, of the plurality of apertures (14) are aligned axially along the length of the tubular wall and spaced circumferentially about the tubular wall.

14. The drug delivery reservoir of claim 4, further comprising a fluid beneficial agent in the reservoir, the fluid beneficial agent having a volume and a concentration increasing from a region proximate the outlet end to a region proximate the distal end, wherein the dip tube (13) is configured to deliver the volume of the fluid beneficial agent at a substantially uniform concentration.

15. The drug delivery reservoir of claim 1, further comprising a junction with a first dip tube section and a second dip tube section each extending from an outlet thereof.
